# FASCICULE DE BREVET EUROPEEN

(11) **EP 2 252 575 B1**
(45) Date de publication et mention de la délivrance du brevet: **19.12.2012**
(21) Numéro de dépôt: 09720924.1
(22) Date de dépôt: 30.01.2009
(51) Int. Cl.: C07C 217/48, C07C 219/22, A61K 31/135, A61K 31/24

(54) **DERIVES DE 2-AMINO-2-PHENYL-ALKANOL, LEUR PREPARATION ET LES COMPOSITIONS PHARMACEUTIQUES QUI LES CONTIENNENT**
2-AMINO-2-PHENYLALKANOLDERIVATE, DEREN HERSTELLUNG UND PHARMAZEUTISCHE ZUSAMMENSETZUNGEN, DIE DIESE ENTHALTEN
2-AMINO-2-PHENYLALKANOL DERIVATIVES, PREPARATION THEREOF AND PHARMACEUTICAL COMPOSITIONS CONTAINING SAME

(30) Priorité: 31.01.2008 FR 0800521; 30.07.2008 US 84829
(43) Date de publication de la demande: 24.11.2010
(73) Titulaire: Oroxcell, 93230 Romainville (FR); Denis, Alexis, 75011 Paris (FR)
(72) Inventeur: DENIS, Alexis, F-75011 Paris (FR); PACHOT, Jean, F-94210 La Varenne (FR); DINI, Christophe, F-91220 Le Plessis-Pâte (FR)
(74) Mandataire: Hirsch & Associés
(86) Numéro de dépôt international: PCT/FR2009/000106
(87) Numéro de publication internationale: WO 2009/112703

(56) Documents cités:
- EP-A- 1 110 549
- WO-A-2007/140611
- GB-A- 1 434 826
- MARTIN, ARNAUD ET AL: "Synthesis of 2-(methylamino)-2-phenylbutyl 3,4,5-trimethoxybenzoate, the main bioactive metabolite of trimebutine maleate" ARZNEIMITTEL-FORSCHUNG , 50(6), 544-549 CODEN: ARZNAD; ISSN: 0004-4172, 2000, XP001538217
- DATABASE CA [Online] CHEMICAL ABSTRACTS SERVICE, COLUMBUS, OHIO, US; FIAUX, HELENE ET AL: "Pyrrolidine derivatives as new inhibitors of .alpha.-mannosidases and growth inhibitors of human cancer cells" XP002478241 extrait de STN Database accession no. 2006:503079 -& CHIMIA , 60(4), 185-189 CODEN: CHIMAD; ISSN: 0009-4293, 2006, XP002478240

## Description

La présente invention concerne des dérivés de 2-amino-2-phényl-alkanol diversement substitués qui sont particulièrement intéressants notamment pour leur action analgésique. La présente invention concerne également la préparation de ces dérivés ainsi que les compositions pharmaceutiques qui les contiennent.

Dans la demande internationale WO 99/01417 a été décrit le (S) 3,4,5-triméthoxy benzoate de 2-méthylamino-2-phényl-n.butyle et son utilisation dans le traitement de la douleur chronique.

Dans la demande européenne EP 1 110 549 l'utilisation de la trimébutine [maléate de 3,4,5-triméthoxy benzoate de (2-méthylamino-2-phényl)butyle] ou ses stéréoisomères dans le traitement des troubles inflammatoires et de la douleur ont été décrits.

Dans la demande de brevet britannique GB 1 434 826 ont été décrits des esters d'aminoalcools de structure : 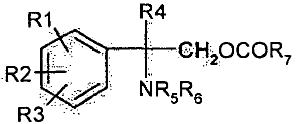 dans laquelle R1 à R3 peuvent être notamment un atome d'hydrogène, R4 peut être un radical alkyle, R7 peut être aryle éventuellement substitué par 1 à 3 radicaux alkoxy et R5 et R6 représentent un atome d'hydrogène, un radical alkyle ou aralkyle ou forment ensemble avec l'atome d'azote auquel ils sont attachés un hétérocycle. Les produits sont utiles comme agents anti-spasmodiques. La demande britannique décrit également des carbamates pour lesquels R₇ possède la structure -NH-R"₇. Les arylcarbamates ainsi constitués sont doués d'activité analgésique et anti-inflammatoire. Cependant les modifications apportées sur l'amine étaient assez limitées et ne pouvaient pas conduire à des analgésiques puissants.

La publication de Martin et al, Arzneilmittel forschung, 50(1), 544-549 (2000) décrit la synthèse de méthylamino-2-phényl-2-butyl-3,4,5-triméthoxybenzoate (desméthyltrimé-butine). Le Schéma 4 décrit un essai de synthèse par triméthoxybenzoylation d'aminoalcool N-formylé et conduit à l'intermédiaire de formule XIII dont la structure est N-formylée. Aucune donnée pharmaceutique n'est signalée.

Il a maintenant été trouvé que des esters dérivés de 2-amino-2-phényl-alkanol de formule générale : 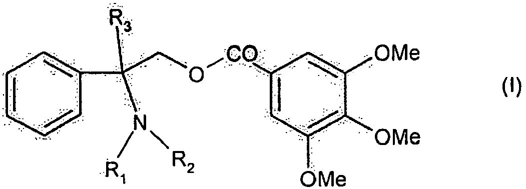 dans laquelle :
R₁ est un atome d'hydrogène, un radical alkyle contenant 1 à 4 atomes de carbone en chaîne droite ou ramifiée, un radical alkyle contenant 2 à 4C en chaîne droite ou ramifiée substitué par hydroxy, alkoxy, alkylthio, acyloxy, amino, alkylamino, dialkylamino, alkylcarbamoyloxy, alkoxycarbonylamino, uréido ou alkyluréido,
R₂ est un radical -CO-R dans lequel R est un radical alkyle, un radical aryle, hétérocyclyle, benzyle ou hétérocyclylméthyle,
un radical -CO-Y-R₄ pour lequel Y est un hétéroatome choisi parmi -O-, -S-, -NH-, -Nalk-pour lequel alk est un radical alkyle droit ou ramifié contenant 1 à 4C, et R₄ est choisi parmi les radicaux alkyle, aryle, aralkyle ou hétérocyclylalkyle, pouvant être substitués par un ou plusieurs atomes d'halogène ou radicaux hydroxy, alkyle contenant de 1 à 4C en chaîne droite ou ramifiée, alkoxy, alkylthio, acylaminoalkylthio, alkoxycarbonyle ou acylamino dont les restes alkyle contiennent 1 à 4C en chaîne droite ou ramifiée, ou oxo, ou pouvant être substitués par un radical R₅COO- dans lequel R₅ est un radical alkyle éventuellement substitué par benzyloxycarbonylamino, acylamino ou par le reste d'un aminoacide, ou représente un radical hétérocyclyle, ou bien
R₂ est un radical alkyle contenant 2 à 4C substitué par hydroxy, alkoxy, alkylthio, acyloxy, amino, alkylamino, dialkylamino dont les parties alcoyle peuvent former avec l'atome d'azote auquel elles sont attachées, un hétérocycle à 5 ou 6 chaînons portant éventuellement un autre hétéroatome choisi parmi l'oxygène ou l'azote, ou substitué par alkylcarbamoyloxy, alkoxycarbonylamino, uréido ou alkyluréido, étant entendu que ledit radical alkyle substitué est en chaîne droite ou ramifiée et comporte au moins 2 atomes de carbone entre l'atome d'azote portant R₂ et le substituant ;
R₃ est un radical alkyle contenant 1 à 4 atomes de carbone en chaîne droite ou ramifiée,
sous leur formes R ou S ou leurs mélanges, ainsi que leurs sels pharmaceutiquement acceptables, lorsqu'ils existent, présentent une activité particulièrement intéressante comme analgésiques, notamment dans le traitement de la douleur chronique.

Il est entendu que, sauf mention spéciale, les radicaux ou les restes alkyle ou acyle sont droits ou ramifiés et contiennent 1 à 7 atomes de carbone, notamment les radicaux acyle peuvent être des radicaux acétyle. Les radicaux aryle ou aralkyle peuvent être des radicaux mono ou bicycliques, comprenant 6 à 10 chaînons, par exemple phényle, naphtyle, benzyle, phénéthyle ou naphtylalcoyle.

Il est entendu que les radicaux hétérocyclyle peuvent être des radicaux mono ou bicycliques aromatiques ou non, comprenant 5 à 10 chaînons et contenant 1 à 4 hétéroatomes choisis parmi l'oxygène, l'azote ou le soufre. Notamment ils peuvent être choisis parmi les radicaux thiényle, furyle, pyrrolyle, pyrrolidinyle pipéridyle pyridyle, pyrazinyle, pyrimidinyle, pyridazinyle, pipérazinyle, dioxolyle, imidazolyle, imidazolinyle, pyrazolyle, tétrazolyle, pyrannyle, tétrahydropyrannyle, tétrahydrofuranyle, oxazolyle, thiazolyle, thiazinyle, morpholinyle, thiomorpholinyle, indolyle, indolizinyle, quinolyle, naphtyridinyle.

Il est entendu que les amino-acides cités ci-avant peuvent être notamment choisis parmi glycine, alanine, leucine, isoleucine, proline, valine, phénylalanine en série L ou D et que ces groupements sont protégés préalablement aux réactions de synthèse, sous forme d'amides ou de carbamates ; la protection et la libération des radicaux protecteurs s'effectue selon les méthodes décrites par T.W. Greene et P.G.M. Wuts, Protective Groups in Organic Synthesis, 4th Edition ISBN 978-0-471-69754-1, December 2006.

Les atomes d'halogène sont choisis parmi le chlore, le fluor, le brome et l'iode.

Selon un mode préféré de réalisation de l'invention, les radicaux alkyle ou acyle sont droits ou ramifiés et contiennent 1 à 4 atomes de carbone.

Selon l'invention, les esters dérivés de 2-amino-2-phényl-alkanol de formule générale (I) sont préparés par action d'un dérivé de formule générale : 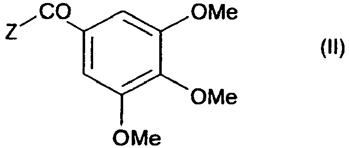 dans laquelle Z est un atome d'halogène, un radical hydroxy ou le reste d'un ester réactif, sur le dérivé de 2-amino-2-phényl alkanol, de formule générale : 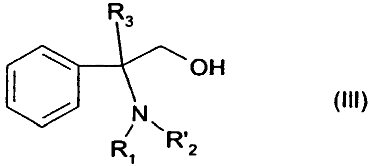 dans laquelle R₁ et R₃ sont définis comme précédemment et R'₂ est un atome d'hydrogène ou est défini comme R₂ précédemment, suivie le cas échéant, lorsque l'un de R'₂ ou R₁ est l'atome d'hydrogène, de la substitution de l'amine de l'ester dérivé de 2-amino-2-phényl-alkanol obtenu, de formule générale : 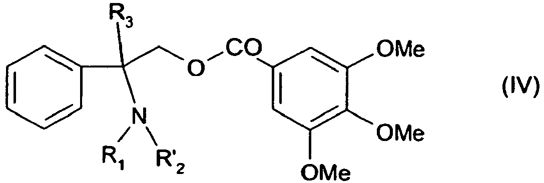 dans laquelle R₁, R'₂ et R₃ sont définis comme ci-dessus,
- soit, lorsque R'₂ est H, et si l'on veut obtenir des dérivés pour lesquels R₂ est -CO-R, par action d'un dérivé réactif de l'acide de formule générale

   R-COOH (V)

   dans laquelle R est défini comme précédemment,
- soit, lorsque R'₂ est H, et si l'on veut obtenir des dérivés pour lesquels R₂ est -CO-Y-R₄, Y étant O, S, NH ou Nalk
   - ou bien, par action du phosgène, suivie de la réaction avec l'alcool, le thiol ou l'amine de formule générale :

      R₄-YH (VI)

      dans laquelle R₄ est un radical alkyle éventuellement substitué et dont le cas échéant les fonctions altérables dans la réaction sont préalablement protégées, ou un radical aryle, aralkyle ou hétérocyclylalkyle, et Y est l'atome d'oxygène ou de soufre, ou un radical NH ou Nalk
   - ou bien, par action de l'halogénure de formule générale :

      R₄-Y-COHal (VII)

      dans laquelle R₄ est défini comme précédemment, de préférence aryle ou alkyle ramifié, Y est l'atome d'oxygène ou de soufre et Hal est un atome d'halogène, de préférence le chlore,
   - ou bien, lorsque l'on veut obtenir un radical R₄ portant la substitution -C(alk)-O-CO-R₅, définie ci-après, pour laquelle alk est un radical alcoyle contenant 1 à 6 atomes de carbone en chaîne droite ou ramifiée et R₅ est défini comme dans la formule générale (I), par action du chloroalkylchloroformate, suivie de la réaction du produit obtenu avec un sel alcalin de l'acide correspondant R₅COOH, par exemple le sel de sodium, de potassium ou le sel de césium R₅COOCs, ou encore le sel d'argent ou un sel d'ammonium quaternaire (comme par exemple le sel de tert-butyl ammonium), de cet acide.
- soit, lorsque R'₂ est H, et si l'on veut obtenir des dérivés pour lesquels R₂ est alkyle substitué, ou bien lorsque l'on a obtenu un dérivé de formule générale (IV) pour lequel R₁ est un atome d'hydrogène et R'₂ est défini comme R₂, et si l'on veut obtenir un produit de formule générale (I) pour lequel R₁ est alkyle éventuellement substitué, par acylation par un halogénure d'acide ou un ester réactif de structure :

   R₂-CO-Z (VIIIa)

   ou

   R₁-CO-Z x (VIIIa)

   dans laquelle R₁ ou R₂ sont définis comme ci-dessus et Z est un atome d'halogène ou le reste d'un ester réactif, suivie de la réduction de l'amide formé en une amine.
- soit encore, lorsque l'on a obtenu un dérivé de formule générale (IV) pour lequel R₁ est un atome d'hydrogène et R'₂ est défini comme R₂, et si l'on veut obtenir un produit de formule générale (I) pour lequel R₁ est alkyle, par action d'un dérivé halogéné de formule

   R₁-X (IX)

   dans laquelle R₁ est un radical alkyle et X est un atome d'halogène ou un radical sulfonique, en présence d'une base.

Le produit de formule générale (II) peut être un dérivé réactif de l'acide 3,4,5-triméthoxy benzoïque comme un halogénure d'acide ou un ester réactif.

La réaction du dérivé de 2-amino-2-phényl alkanol de formule générale (III) s'effectue de préférence au moyen d'un dérivé pour lequel R'₂ est l'atome d'hydrogène.

Lorsque le produit de formule générale (II) est un dérivé réactif de l'acide 3,4,5-triméthoxy benzoïque comme l'halogénure d'acide ou un ester réactif, la réaction du dérivé de formule générale (II) sur le dérivé 2-amino-2-phényl alkanol de formule générale (III) s'effectue avantageusement en présence d'une base azotée comme par exemple la triéthylamine, la diméthylaminopyridine, diisopropyléthylamine dans le cas de l'halogénure d'acide de formule (II) et la réaction s'effectue généralement dans un solvant organique comme un solvant chloré (dichlorométhane, dichloréthane, chloroforme par exemple), à une température comprise entre 0 et 70°C, de préférence en opérant sous azote. Et dans le cas d'un ester réactif de formule (II), en présence du méthylate de sodium dans un solvant organique comme le toluène en présence d'un alcool comme le méthanol ou l'éthanol, à une température comprise entre 25 et 150°C.

Lorsque Z est un atome d'halogène, il est avantageusement choisi parmi le chlore ou le brome.

Lorsque le produit de formule générale (II) est l'acide 3,4,5-triméthoxy benzoïque la réaction s'effectue généralement en présence d'un carbodiimide, dans un solvant halogéné (dichlorométhane, dichloréthane, chloroforme par exemple), à une température comprise entre 0 et 70°C.

Il est entendu que lorsque l'on veut obtenir un dérivé de formule générale (IV) de forme R ou S, on fait agir un dérivé de 2-amino-2-phényl alkanol, de formule générale (III) de forme R ou S. Il est également entendu que les dérivés de formule générale (IV) de forme R ou S conduisent à des dérivés de formule générale (I) de forme R ou S.

La substitution de l'amine du dérivé de formule générale (IV) par action d'un dérivé réactif de l'acide de formule générale (V) s'effectue avantageusement au moyen de l'halogénure d'acide ou d'un ester, ester réactif notamment, de préférence en présence d'un agent de condensation comme une amine tertiaire (triéthylamine, diisopropylethylamine, diméthylaminopyridine notamment). La réaction s'effectue généralement dans un solvant organique comme un solvant chloré (dichlorométhane, dichloréthane, chloroforme par exemple), à une température comprise entre 0 et 70°C. Lorsque l'on souhaite obtenir le dérivé pour lequel R₂ est formyle, on opère avantageusement par action d'un ester, l'utilisation d'un solvant peut s'avérer non indispensable.

La réaction de l'alcool ou du thiol de formule générale (VI) s'effectue après action du phosgène sur l'amine du dérivé de formule générale (IV), (sous forme d'une solution dans un solvant aromatique comme par exemple le toluène), dans un solvant organique comme un solvant halogéné (par exemple solvant chloré comme le dichlorométhane, dichloréthane ou le chloroforme) en présence d'une amine tertiaire (triéthylamine, diisopropyléthylamine, diméthylaminopyridine notamment) à une température comprise entre 0 et 25°C. La réaction de l'alcool ou du thiol de formule générale (VI) est réalisée par addition du dérivé de formule générale (VI) en présence d'une amine tertiaire comme cité ci-dessus, à une température comprise entre 0 et 70°C, dans un solvant halogéné (dichlorométhane, dichloréthane, chloroforme par exemple). De préférence on opère sous azote. Il est entendu que lorsque des substituants risquent d'être altérés au cours de la réaction, ces derniers sont préalablement protégés. La protection, et la libération des radicaux protecteurs s'effectue selon les méthodes décrites par T.W. Greene et P.G.M. Wuts, Protective Groups in Organic Synthesis, 4th Edition ISBN 978-0-471-69754-1, December 2006.

La réaction du dérivé de formule générale (VII) sur l'amine du dérivé de formule générale (IV) s'effectue en présence d'un agent de condensation comme une amine tertiaire (triéthylamine, diisopropylethylamine, diméthylaminopyridine notamment). La réaction s'effectue généralement dans un solvant organique comme un solvant chloré (dichlorométhane, dichloréthane, chloroforme par exemple) ou le tétrahydrofurane, à une température comprise entre 0 et 70°C. De préférence on opère sous azote.

Lorsque l'on veut obtenir un produit pour lequel le radical R₄ porte la substitution -C(alk)-O-CO-R₅, on opère par action du chloroalkylchloroformate sur le produit de formule générale (IV), la réaction s'effectue dans un solvant organique comme un solvant chloré (dichlorométhane, dichloréthane par exemple), ou comme un éther (tétrahydrofuranne par exemple), à une température comprise entre -10 et 50°C. Elle est suivie de la réaction du produit obtenu avec un sel alcalin de l'acide correspondant R₅COOH, par exemple le sel de sodium, de potassium ou le sel de césium, le sel d'argent, ou un sel d'ammonium quaternaire, dans un solvant organique comme par exemple un amide comme le diméthylformamide, un solvant chloré (dichlorométhane par exemple), un ester (acétate d'éthyle par exemple), un hydrocarbure aromatique (toluène par exemple), un nitrile (acétonitrile par exemple), une cétone (acétone, méthyl ethyl cétone par exemple), en présence ou en absence d'iodure de sodium, à une température comprise entre 0 et 60°C.

Lorsque l'on veut obtenir un dérivé de formule générale (I) pour lequel R2 est alkyle substitué ou pour lequel R1 est alkyle éventuellement substitué, laréaction d'alkylation de l'amine du dérivé de formule générale (IV) s'effectue dans un solvant halogéné (dichlorométhane, dichloréthane par exemple) ou dans un éther (tétrahydrofurane), à une température comprise entre 0 et 70°C. Le cas échéant l'ester réactif peut être préparé au moyen d'hydroxybenzotriazole. La réduction s'effectue en présence de borane dans le tétrahydrofurane, à une température comprise entre 0 et 70°C.

La réaction du produit de formule (IX) sur le dérivé de 2-amino-2-phényl-alkanol de formule générale (IV), s'effectue au moyen d'un dérivé halogéné pour lequel l'halogène est choisi parmi le chlore, le brome ou l'iode ou au moyen d'un dérivé sulfonique comme le tosylate, le mésylate ou le triflate, en présence d'une base comme un carbonate alcalin (NaHCO₃ ou KHCO₃ par exemple).

Les dérivés de l'acide 3,4,5-triméthoxy benzoïque de formule générale (II) peuvent être préparés selon les méthodes habituelles de transformation des acides carboxyliques en leurs dérivés réactifs, qui n'altèrent pas le reste de la molécule.

Les dérivés de formule générale (III) peuvent être préparés selon la méthode décrite dans les demandes de brevet FR 2 765 218 ou EP 510 168, ou par analogie avec la méthode décrite dans ces demandes.

Les dérivés halogénés de formule générale (VII) peuvent être préparés par action du phosgène sur l'alcool ou le thiol correspondant de formule générale (VI).

On opère dans des conditions analogues aux conditions décrites précédemment pour l'action du phosgène sur l'amine du dérivé de formule générale (IV).

Il est entendu que lorsque l'on veut obtenir un produit de formule générale (I) sous forme S ou R, on fait agir un dérivé de 2-amino-2-phényl alkanol de formule générale (III) de forme S ou R.

Les dérivés de 2-amino-2-phényl alkanol de formule générale (III) de forme S ou R peuvent être préparés selon la méthode décrite dans le brevet européen EP 510,168 ou par séparation selon les méthodes habituelles de séparation des énantiomères qui n'affectent pas le reste de la molécule.

Lorsqu'ils existent les sels pharmaceutiquement acceptables peuvent être des sels d'addition avec les acides. Notamment des sels avec les acides minéraux comme par exemple les chlorhydrates, les bromhydrates, les sulfates, les phosphates ou les sels d'addition avec les acides organiques comme par exemple les acétates, les maléates, les fumarates, les tartrates, les citrates.

Les dérivés de formule générale (I) peuvent être purifiés selon les méthodes habituelles, notamment par chromatographie ou par cristallisation.

Les dérivés de formule générale (I) sont particulièrement intéressants du fait de leur activité analgésique puissante, notamment dans la douleur chronique.

Leur activité a été mise en évidence in vitro dans le test de l'inhibition des canaux sodiques par application de la méthode de G. B. Brown, 3H-batrachotoxinin-A benzoate binding to voltage-sensitive sodium channels : inhibition by the channel blockers tetrodotoxin and saxitoxin, J. Neurosci., 6, 2064 (1986). In vitro dans ce test, les produits selon l'invention ont manifesté des activités entre 25 et 90 % d'inhibition pour des concentrations de 3,2 (M).

Par ailleurs, in vivo leur activité a été mise en évidence chez le rat dans le test de la douleur provoquée par la formaline, phase courte et phase longue adaptée de la méthode de Wheeler-Aceto et coll., psychopharmacology, 104, 35-44 (1991). Dans cette méthode le produit de l'exemple 4 s'est montré actif à la dose de 39,3 mg/kg par voie sous-cutanée en phase courte et en phase longue. L'activité in vivo a été également mise en évidence dans le test de douleur abdominale, par irritation et distension du colon, chez le rat selon la méthode adaptée de la méthode décrite par Langlois et coll., Eur. J. Pharmacol., 324, 211-217 (1997). Dans ce test le produit de l'exemple 4 s'est montré actif chez le rat, à partir de 13,1 mg/kg et à des doses de 26,2 mg/kg par voie sous-cutanée.

De plus, il a été mis en évidence, après injection i.v. chez le rat, que le temps de demi-vie de certains produits selon la présente invention sont particulièrement élevés.

Enfin les produits selon l'invention ne manifestent pas de toxicité. En effet, chez la souris par voie intrapéritonéale à des doses de 26,2 mg/kg en 7 administrations répétées en 2 jours et chez le rat par voie orale à 39,3 mg/kg, aucune mortalité et aucun signe de comportement anormal n'ont été observés.

Particulièrement intéressants sont les produits de formule générale (I), désignés (Ia), pour lesquels R₁ et R₃ sont définis comme précédemment et R₂ dans -NR₁R₂ est un radical -CO-O-R₄ pour lequel R4 a une structure :

-C(alk)-O-CO-R₅ (X)

alk étant un radical alcoyle contenant 1 à 6 atomes de carbone en chaîne droite ou ramifiée et R₅ est défini comme dans la formule générale (I), ou bien, désignés (Ia'), pour lesquels R₃ est défini comme précédemment et dans -NR₁R₂, R₁ est un atome d'hydrogène et R₂ est un radical -CO-R tel que défini précédemment.

Et parmi les produits de formule générale (Ia) et (Ia') sont plus particulièrement préférés les produits de formule générale (Ia) pour lesquels R₁ est un atome d'hydrogène, un radical alkyle contenant 1 à 4 atomes de carbone (notamment méthyle) ou un radical 2-méthoxyéthyle.

Egalement préférés sont les produits de formule générale (I), désignés (Ib), pour lesquels R₁ et R₃ sont définis comme précédemment et R₂ dans -NR₁R₂ est un radical alkyle substitué tel que défini précédemment pour R₂ dans la formule générale (I).

Les exemples suivants illustrent la présente invention.

Dans les exemples qui suivent, les abréviations employées ont la signification suivante :
DMF diméthylformamide
DMSO diméthylsulfoxyde
THF tétrahydrofurane
DIPEA N,N-diisopropyléthylamine
CCM chromatographie en couche mince

### Exemple 1 ORC012

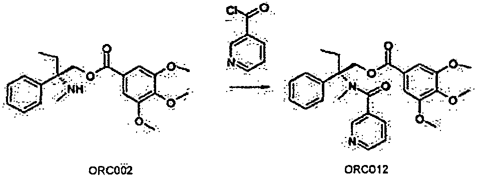

0,300g (0,8 mmol, 1 éq.) de 3,4,5-triméthoxybenzoate de (S) 2-méthylamino-2-phényl-n-butyle et 0,442g (2,4 mmol, 3 éq.) de nicotinoyl chloride hydrochloride sont placés sous azote puis mis en suspension dans 3ml de 1,2-dichloroethane sec. On additionne alors 0,4 ml (2,4 mmol, 3 éq.) de N,N-diisopropyléthylamine. L'agitation est maintenue 20 heures à température ambiante.

Le mélange réactionnel est traité avec 6 ml de NaHCO₃ saturé puis extrait avec 12 ml de dichlorométhane. La phase organique est de nouveau lavée avec 6 ml NaHCO₃ saturé puis séchée sur Na₂SO₄, filtrée et évaporée à sec. Le résidu obtenu est purifié par chromatographie flash sur gel de silice (gradient acétate d'éthyle/cyclohexane 3:7 à 6:4, v/v) pour donner 0,189 g (46 %) du produit attendu 3,4,5-triméthoxybenzoate de (S) 2-(méthyl 3-pyridylcarbonyl amino)-2-phényl-n-butyle sous forme d'une mousse blanche.
¹H-RMN (CDCl₃, 400MHz) :
δ (ppm)= 0,93 (t, J=7,6Hz, 3H, CH₃) ; 2,27 (m, 1H, CH₂ diastéréotope) ; 2,52 (m, 1H, CH₂ diastéréotope) ; 2,91 (s, 3H, NCH₃) ; 3,77 (s, 6H, 2xOCH₃) ; 3,82 (s, 3H, OCH₃) ; 5,03 (d, J=11,5Hz, 1H, OCH₂) ; 5,24 (d, J=11,5Hz, 1H, OCH₂) ; 7,10-7,40 (m, 8H, ArH) ; 7,73 (d, J=7,8Hz, 1H, ArH) ; 8,57 (d, J=4,9Hz, 1H, ArH) ; 8,66 (m, 1H, ArH). LC-MS (ES): m/z= 479 (M+H)⁺. R_{f} (SiO₂, dichlorométhane/méthanol 98:2): 0,36.

Le 3,4,5-triméthoxybenzoate de (S) 2-méthylamino-2-phényl-n-butyle peut être préparé selon la méthode décrite dans les demandes FR 2765218 et EP 0510168.

### Exemple 2 Synthèse d'ORC011 :

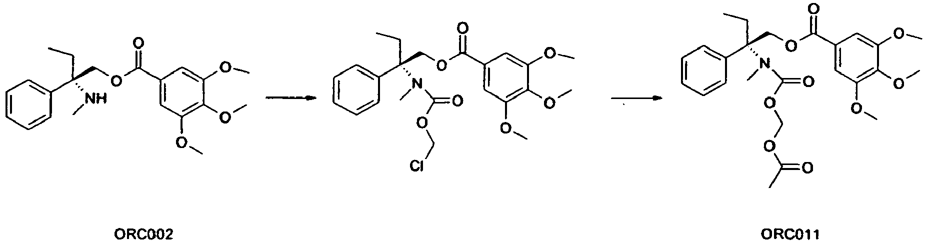

### Etape 1 :

1,17g (2,83 mmol ; 1 éq.) de 3,4,5-triméthoxybenzoate de (S) 2-méthylamino-2-phényl-n-butyle est mis sous azote puis solubilisé dans 1 ml de 1,2-dichloroéthane. On additionne ensuite lentement, goutte à goutte, 0,77 ml (8,50 mmol ; 3 éq.) de chlorométhyl chloroformate. On agite pendant 6 heures à température ambiante puis le mélange réactionnel est traité avec 10 ml NaHCO₃ saturé puis extrait avec 20 ml de dichlorométhane. La phase organique est de nouveau lavée avec 10 ml NaHCO₃ saturé, séchée sur Na₂SO₄, filtrée puis évaporée à sec.

Le résidu obtenu est purifié par chromatographie flash sur gel de silice (acétate d'éthyle/cyclohexane 2:8, v/v) pour donner 0,830g (63 %) du produit attendu 3,4,5-triméthoxybenzoate de (S) 2-(chlorométhoxycarbonyl méthyl amino)-2-phényl-n-butyle sous forme d'une huile incolore.

¹H-RMN (CDCl₃, 400MHz) : δ (ppm)= 0,92 (t, J=7,2Hz, 3H, CH₃) ; 2,24 (m, 1H, CH₂ diastéréotope) ; 2,45 (m, 1H, CH₂ diastéréotope) ; 3,03 (s(1), 3H, NCH₃) ; 3,89 (s, 6H, 2xOCH₃) ; 3,91 (s, 3H, OCH₃) ; 4,99 (s(1), 2H, OCH₂) ; 5,71 (s(1), 2H, ClCH₂) ; 7,19-7,37 (m, 7H, ArH). MS (CI, NH₃) : m/z (%) = 483 ((M+NH₄)⁺, 65], 465 [(M^{+.}), 10], 343 [(MH-C₃O₂NClH₅)⁺, 100]. R_{f}(SiO₂, acétate d'éthyle/cyclohexane, 3:7) : 0,41

### Etape 2

0,289g (0,62 mmol ; 1 éq.) de 3,4,5-triméthoxybenzoate de (S) 2-(chlorométhoxycarbonyl méthyl amino)-2-phényl-n-butyle sont mis en solution dans 10,2 ml de N,N-diméthylformamide puis placé à 0°C. On additionne alors lentement une suspension blanchâtre de 0,125 g (0,62 mmol ; 1 éq.) d'acétate de césium dans 4,1ml de N,N-diméthylformamide. On maintient l'agitation 17 heures à température ambiante.

On additionne alors 0,038 g (0,18 mmol ; 0,3 éq.) supplémentaire d'acétate de césium et on poursuit l'agitation 7 heures à température ambiante.

Le mélange réactionnel est alors dilué dans 20 ml d'acétate d'éthyle. La phase organique est lavée avec 2x12 ml de NaHCO₃ (10 %), 2x12 ml d'eau et 12 ml de NaCl saturé, séchée sur Na₂SO₄, filtrée et évaporée à sec.

Le résidu obtenu est purifié par chromatographie flash sur gel de silice (gradient acétate d'éthyle/cyclohexane 1:9 à 3:7, v/v) pour donner 0,065 g (20 %) du produit attendu 3,4,5-triméthoxybenzoate de (S) 2-(acétoxyméthoxycarbonyl méthyl amino)-2-phényl-n-butyle sous forme d'une huile incolore.

¹H-RMN (CDCl₃, 400MHz) : δ (ppm)= 0,80 (t, J=6,5Hz, 3H, CH₃) ; 1,92 (s, 3H, CH₃CO) ; 2,13 (s(1), 1H, CH₂ diastéréotope) ; 2,35 (m, 1H, CH₂ diastéréotope) ; 2,93 (s(1), 3H, NCH₃) ; 3,80 (s, 6H, 2xOCH₃) ; 3,82 (s, 3H, OCH₃) ; 4,82 (d, J=10,6Hz, 1H, CH₂ diastéréotope) ; 4,96 (m, 1H, OCH₂ diastéréotope) ; 5,57 (s(1), 2H, OCH₂O) ; 7,10-7,27 (m, 7H, ArH). MS (CI, NH₃) : m/z (%)= 479 [(M+NH₄-C₂H₅)⁺, 100], 285 [15], 230 [15].

R_{f}(SiO₂, acétate d'éthyle/cyclohexane 3:7):0,21.

### Exemple 3 Synthèse d'ORC007 :

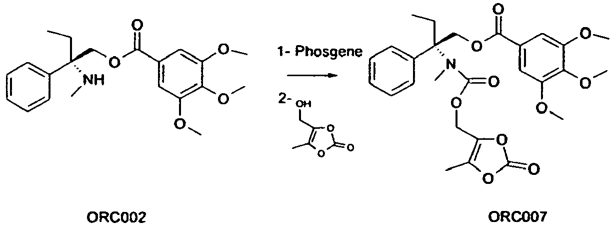

0,40 g (1,07 mmol ; 1éq.) de 3,4,5-triméthoxybenzoate de (S) 2-méthylamino-2-phényl-n-butyle est placé sous azote puis mis en solution dans 53 ml de dichlorométhane. On additionne alors 1,3 ml (2,53 mmol ; 2,36 éq.) d'une solution à 20 % dans le toluène de phosgène puis 0,16 ml (1,18 mmol ; 1,1éq.) de triéthylamine. On maintient l'agitation 22 heures à température ambiante. On additionne ensuite 0,69g (5,35 mmol ; 5éq.) de 4-(hydroxyméthyl)-5-méthyl-1,3-dioxol-2-one. Après 24 heures supplémentaires à température ambiante, le mélange réactionnel est lavé avec 2x40 ml d'eau et 1x40ml HCl 1 M. La phase organique est séchée sur Na₂SO₄, filtrée puis évaporée à sec.

Le résidu obtenu est purifié par 2 chromatographies flash sur gel de silice (dichlorométhane puis gradient acétate d'éthyle/cyclohexane 1 :9 à 3 :7, v/v) pour donner 0,085g (15 %) du produit attendu 3,4,5-triméthoxybenzoate de (S) 2-(5-méthyl-1,3-dioxol-2-one-4-yl méthyl amino)-2-phényl-n-butyle sous forme d'une huile incolore.

¹H-RMN (CDCl₃, 400MHz) :
δ(ppm)= 0,78 (t, J=7,2Hz, 3H, CH₃) ; 1,94 (s, 3H, CH₃) ; 2,02 (m, 1H, CH₂ diastéréotope) ; 2,32 (m, 1H, CH₂ diastéréotope) ; 3,02 (s(1), 3H, NCH₃) ; 3,80 (s, 6H, 2xOCH₃) ; 3,82 (s, 3H, OCH₃) ; 4,64 (s(1), 2H, OCH₂Csp₂) ; 4,80 (m, 1H, OCH₂ diastéréotope) ; 4,93 (m, 1H, OCH₂ diastéréotope) ; 7,10-7,27 (m, 7H, ArH).
MS (CI, NH₃) : m/z (%) = 547 ((M+NH₄)⁺, 5], 479 [80], 260 [100].
R_{f}(SiO₂, dichlorométhane/méthanol, 98:2) : 0,16.

### Exemple 4 Synthèse de ORC020

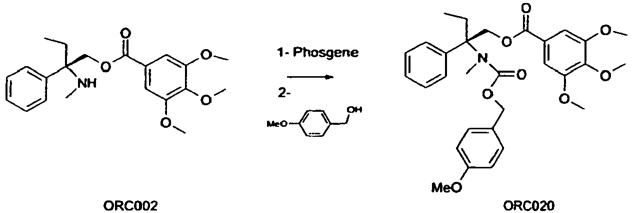

### Etape 1 :

Dans un tricol de 100 ml, sous courant d'azote, 3,4,5-triméthoxybenzoate de (S) 2-méthylamino-2-phényl-n-butyle (1,49 g ; 4,0 mmoles ; 1 éq.) est mis en solution dans 40 ml dichlorométhane. La solution est refroidie à 0°C. Une solution de phosgène dans le toluène à 20 % (4,95 ml ; 9,4 mmoles ; 2,4 éq.) puis N,N-diisopropylethylamine (730 µl ; 4,4 mmoles ; 1,1 éq.) sont additionnés sans dépasser 5°C. Le bain de glace est retiré et le mélange est agité pendant 20 heures. On obtient le 3,4,5-triméthoxybenzoate de (S) 2-(chloroformyl méthyl amino)-2-phényl-n-butyle.

### Etape 2 :

2,53 ml de 4-méthoxybenzyl alcohol (20 mmoles ; 10 éq.) sont mis en solution dans 10 ml de dichlorométhane, puis la moitié du volume du mélange réactionnel (871,8 mg ; 2 mmoles ; 1 éq.) de 3,4,5-triméthoxybenzoate de (S) 2-(chloroformyl méthyl amino)-2-phényl-n-butyle (étape 1), soit 20 ml de la solution obtenue, sont ajoutés goutte à goutte. Après 1 heure à température ambiante, N,N-diisopropyléthylamine (1,30 ml ; 8 mmoles ; 4 éq.) sont ajoutés puis l'agitation est maintenue pendant 20 heures. Le mélange réactionnel est versé dans 20 ml d'une solution saturée de NH₄Cl (pH=7-8). Après décantation, la phase aqueuse est extraite avec 2x20 ml de dichlorométhane. Les phases organiques sont réunies et lavées avec 2x20 ml d'une solution saturée de NH₄Cl (pH = 7 puis pH = 6), et 20 ml d'une solution de NaCl mi-saturée puis séchée avec Na₂SO₄. Le produit brut est purifié sur gel de silice de (35 parties, éluant acétate d'éthyle/cyclohexane 7/3 pour obtenir une huile transparente de 3,4,5-triméthoxybenzoate de (S) 2-(4-méthoxybenzyloxycarbonyl méthyl amino)-2-phényl-n-butyle (15 % ; 170 mg). L'huile est reprise dans 1 ml d'éther et 1 ml de pentane pour avoir une émulsion qui reste. Le ballon est refroidi pendant 2 heures à 4°C puis le surnageant de solvant est enlevé par une pipette pasteur. Le solide est séché à la pompe à palette pour donner une mousse blanche.
R_{f}(SiO₂, acétate d'éthyle/cyclohexane 7:3): 0,42
¹H-RMN (CDCl₃, 400MHz):
δ (ppm) = 0,86 (t, J = 7,3 Hz, 3H) ; 2,16 (qd, J = 7,3 Hz et 14,8 Hz, 1H, CH₂) ; 2,38 (qd, J = 7,3 Hz et 14,8 Hz, 1H, CH₂) ; 3,09 (sl, 3H) ; 3,80 (s, 3H) ; 3,87 (s, 6H) ; 3,91 (s, 3H) ; 4,93 (ml, 4H) ; 6,79 (m, 2H) ; 7,05 (ml, 1H) ; 7,18 (m, 2H) ; 7,30 (m, 6H).
MS (CI, NH₃): m/z = 555 [(M+NH₄)⁺], 53 7, 494, 463, 268.

### Exemple 5

### Synthèse d'ORC009 :

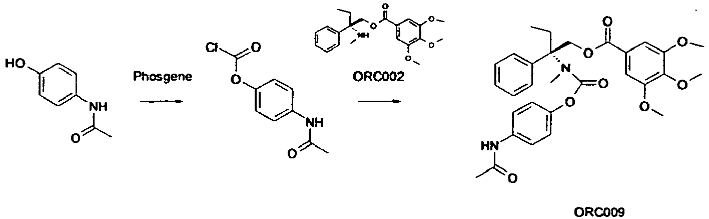

0,081 g (0,54 mmol ; 1 éq.) de 4-acétamidophénol sous azote est mis en suspension dans 3,6 ml d'acétate d'éthyle sec et placé à 0°C. On additionne alors 0,43 ml (0,82 mmol ; 1,54 éq.) d'une solution à 20 % dans le toluène de phosgène. On additionne ensuite (toujours à 0°C) 0,075 ml (0,54 mmol ; 1 éq.) de triéthylamine. On se place ensuite à température ambiante. Après 1 heure, on additionne 0,40 g (1,07 mmol ; 2 éq.) de 3,4,5-triméthoxybenzoate de (S) 2-méthylamino-2-phényl-n-butyle. On poursuit l'agitation 18 heures à température ambiante.

Le mélange réactionnel est dilué dans 5 ml d'acétate d'éthyle puis lavé avec 8 ml d'eau et 8 ml d'HCl 1M. La phase organique est séchée sur Na₂SO₄, filtrée puis évaporée à sec.

Le résidu obtenu est purifié par chromatographie flash sur gel de silice (gradient dichlorométhane à dichlorométhane/méthanol 9 :1, v/v). Le produit obtenu est trituré deux fois dans 1,5 ml de pentane/éther 1:1 pour donner 0,092 g (31 %) d'un solide blanc 3,4,5-triméthoxybenzoate de (S) 2-(4-acétylaminophényloxycarbonyl méthylamino)-2-phényl-n-butyle.
¹H-RMN (CDCl₃, 400MHz): δ (ppm) = 0,86 (t, J=6,8Hz, 3H, CH₃) ; 2,02 (s, 3H, CH₃CO) ; 2,18 (m, 1H, CH₂ diastéréotope) ; 2,43 (m, 1H, CH₂ diastéréotope) ; 3,06 (s(1), 3H, NCH₃) ; 3,79 (s, 6H, 2xOCH₃) ; 3,83 (s, 3H, OCH₃) ; 4,87 (d, J=11,1Hz, 1H, CH₂ diastéréotope) ; 5,03 (m, 1H, CH₂ diastéréotope) ; 6,70 (s(1), 1H, NH) ; 7,14-7,37 (m, 11H, ArH).
MS (CI, NH₃) : m/z (%) = 568 [(M+NH4)⁺, 90], 374 [(MH- C₉O₃NH₈)⁺, 10], 343 [(MH-C₁₀O₃N₂H₁₁)⁺, 40].
R_{f} (SiO₂, dichlorométhane/méthanol 98:2) : 0,28

### Exemple 6 Synthèse d'ORC021

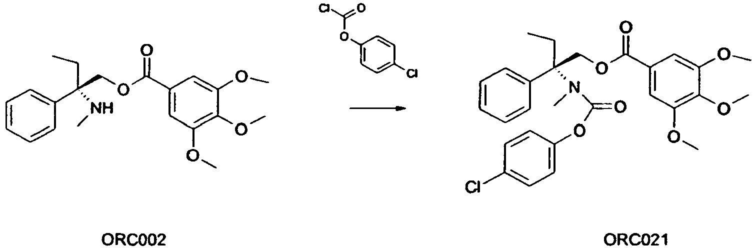

A la solution de 300 mg (0,8 mmoles ; 1 éq.) de 3,4,5-triméthoxybenzoate de (S) 2-méthylamino-2-phényl-n-butyle dans 3 ml de dichlorométhane, 400 µl (2,4 mmoles ; 3 éq.) de DIPEA sont ajoutés goutte à goutte à température ambiante sous azote, puis ensuite 337 µl (2,4 mmoles ; 3 éq.) de 4-chlorophényl chloroformate goutte à goutte. Le mélange est agité à température ambiante pendant 70 heures. Le mélange réactionnel est versé dans 30 ml NaHCO₃ saturée (pH=7), puis extrait avec 30 ml de dichlorométhane, puis 10 ml dichlorométhane. La phase organique est lavée avec 20 ml NaCl saturée (pH=7-8) puis séchée sur Na₂SO₄ et évaporée à sec pour donner une huile. Celle-ci est purification sur colonne de gel de silice (50 partie ; éluant acétate d'éthyle/cyclohexane 2/8). L'huile obtenue est refroidie à -50°C sous azote pour précipiter le produit, 2x2 ml de pentane sont ajoutés. Le produit est ensuite séché sous vide pendant 1 heure pour donner une poudre blanche contenant 4 % de solvant que l'on ne peut évaporer. La poudre est solubilisée dans 1 ml de CH₂Cl₂ puis évaporé à sec pour donner après 20 heures à la pompe une mousse blanche 3,4,5-triméthoxybenzoate de (S) 2-(4-chlorophénoxycarbonyl méthyl amino)-2-phényl-n-butyle (360 mg, 85 %).
R_{f} (SiO₂, acétate d'éthyle/cyclohexane 4:6): 0,66
¹H-RMN (CDCl₃, 400MHz):
δ (ppm) = 0,94 (t, J = 7,4 Hz, 3H) ; 2,27 (m, 1H, CH₂) ; 2,51 (qd, J = 7,4 Hz et 14,6 Hz, 1H, CH₂) ; 3,20 (sl, 3H) ; 3,88 (s, 6H) ; 3,92 (s, 3H) ; 5,00 (dd, J = 10,4 Hz et J = 61,1 Hz, 2H, CH₂) ; 6,80 (sl, 2H) ; 7,23 (ml, 3H) ; 7,29 (m, 2H) ; 7,38 (m, 4H).
MS (CI, NH₃): m/z = 545 [(M+NH₄)⁺], 343, 195.

### Exemple 7 Synthèse d'ORC018:

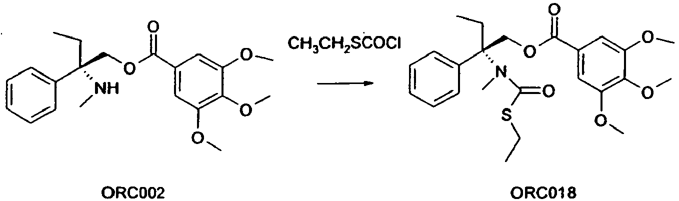

A une solution de 3,4,5-triméthoxybenzoate de (S) 2-méthylamino-2-phényl-n-butyle (300 mg ; 0,8 mmoles ; 1 éq.) dans le dichloroéthane, on ajoute à température ambiante, sous azote, goutte à goutte, la N,N-diisopropyléthylamine (0,4 ml ; 2,4 mmoles ; 3 éq.), puis ensuite goutte à goutte, le chlorothioformate d'éthyle (260 µl ; 2,4 mmoles ; 3 éq.). L'agitation est maintenue à température ambiante pendant 70 heures. Le mélange réactionnel est versé dans 30 ml de NaHCO₃ saturé (pH=7), puis extrait avec 30 ml de dichlorométhane, et 10 ml dichlorométhane. La phase organique est lavée avec 20 ml NaCl saturé (pH=7-8) puis séchée sur Na₂SO₄ et évaporé à sec. L'huile obtenue est dissoute dans 3 ml d'éther, puis sous léger chauffage, 3 ml de pentane sont ajoutés goutte à goutte. La solution trouble est placée à froid pendant 20 heures puis le surnageant est prélevé avec une pipette. L'opération est menée deux fois avec 5 ml éther/pentane. Ensuite les cristaux sont séchés dans le ballon pour obtenir une poudre blanche 3,4,5-triméthoxybenzoate de (S) 2-(éthylthiocarbonyl méthyl amino)-2-phényl-n-butyle (251 mg, 68 %).
R_{f} (SiO₂, acétate d'éthyle/cyclohexane 1:3): 0,50.
¹H-RMN (CDCl₃, 400MHz): δ (ppm) = 0,93 (t, J = 7,4 Hz, 3H) ; 1,24 (t, J= 7,3 Hz, 3H) ; 2,22 (qd, J = 7,4 Hz et 14,3 Hz, 1H, CH₂) ; 2,41 (qd, J = 7,4 Hz et 14,3 Hz, 1H, CH₂) ; 2,83 (q, J = 7,3 Hz, 2H) ; 3,02 (s, 3H) ; 3,88 (s, 6H) ; 3,90 (s, 3H) ; 4,99 (d, J = 11,3 Hz, 1H, CH₂) ; 5,11 (d, J = 11,3 Hz, 1H, CH₂) ; 7,16 (sl, 2H) ; 7,26 (m, 1H) ; 7,34 (m, 4H).
MS (CI, NH₃): m/z = 479 [(M+NH₄)⁺], 462 [(M+H)⁺], 343, 250.

### Exemple 8 ORC033

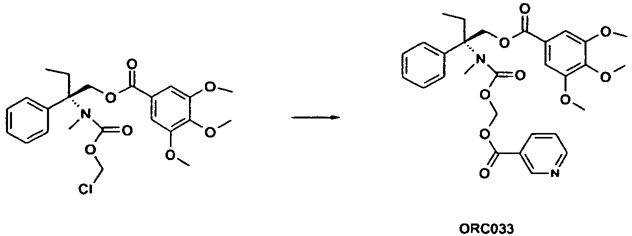

Sous atmosphère d'azote, l'acide nicotinique (0,081 g, 0,00064 mol) est solubilisé dans du DMF (1 mL). Du fluorure de césium (0,098 g, 0,00064 mol) est ajouté et la solution est agitée à température ambiante pendant 15 minutes. La solution est refroidie à 0°C et le 3,4,5-triméthoxybenzoate de (S) 2-(chlorométhoxycarbonyl méthyl amino)-2-phényl-n-butyle (100,0 mg, 0,0002146 mol), préalablement solubilisé dans du DMF (1 mL) est additionné goutte à goutte. Le mélange est chauffé à 70°C pendant 17 heures.

Après refroidissement, de l'acétate d'éthyle (5 mL) est ajouté et la phase organique est lavée avec une solution saturée de NaHCO3 puis une solution saturée de chlorure de sodium. La phase organique est séchée sur Na2SO4, filtrée et évaporée pour donner 120 mg d'huile jaune. Le produit est purifié [(SiO2 ; cyclohexane / AcOEt (3/7)] pour donner 86 mg de 3,4,5-triméthoxybenzoate de (S)-2-(nicotinyloxyméthoxycarbonyl méthyl amino)-2-phényl-n-butyle sous forme d'un solide amorphe blanc.
- CCM : SiO2, cyclohexane / AcOEt (3/7) Rf 0,37
- RMN 1H (CDCl3) : δ 0,86 (3H, t, J=7,55 Hz, CH3-CH2), 2,10-2,22 (1H, m, CH-CH3), 2,40-2,48 (1H, m, CH-CH3), 3,09 (3H, br s, NCH3), 3,85-3,88 (9H, d, J=9,79 Hz, 3 OCH3), 4,88-4,95 (2H, br d, J=9,61 Hz, CH2-O), 5,91 (2H, br s, CH2), 7,14 (2H, s, Harom), 7,25-7,39 (8H, m, Harom), 8,20 (1H, br s, Hpyr), 8,77-8,80 (1H, dd, J1=1,69 Hz, J2=4,89 Hz, Hpyr), 9,15 (1H, br s, Hpyr).
- MS (ES+) : [M+H]+ , m/z : 552

### Exemple 9 ORC035

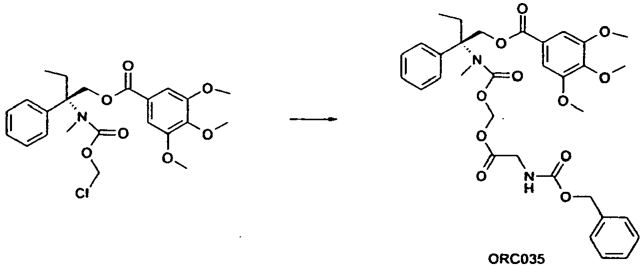

Sous atmosphère d'azote, du N-carbobenzyloxyglycine (0,20 g, 0,00096 mol) est solubilisé dans du DMF (2 mL). Du fluorure de sodium (0,15 g, 0,00096 mol) est ajouté et le mélange est agité pendant 15 minutes à température ambiante.

Le mélange est refroidi à 0°C et du 3,4,5-triméthoxybenzoate de (S) 2-(chlorométhoxycarbonyl méthyl amino)-2-phényl-n-butyle (150,0 mg,0,0003219 mol) en solution dans le DMF (2 mL) est additionné goutte à goutte. Le mélange réactionnel est chauffé à 70°C pendant 2 heures. Après refroidissement, de l'acetate d'éthyle (8 mL) est ajouté et la phase organique est lavée avec une solution aqueuse saturée de bicarbonate de sodium (3 mL), puis une solution de chlorure de sodium saturée (3 mL), puis la phase organique est séchée sur Na2SO4, filtrée puis évaporée pour donner une huile jaune. Le produit est purifié : SiO2, cyclohexane / AcOEt (7/3 puis 6/4) pour donner 197 mg de 3,4,5-triméthoxybenzoate de (S)-2-(benzyloxycarbonylaminoacétoxyméthoxycarbonyl méthyl amino)-2-phényl-n-butyle sous forme d'huile jaune.
CCM : SiO2, cyclohexane / AcOEt (3/7) Rf 0,50
RMN ¹H (CDCl₃) : δ (ppm)= 0,85 (3H, t, J=7,53 Hz, CH₃-CH₂), 2,08-2,27 (1H, m, CH-CH₃), 2,36-2,46 (1H, m, CH-CH₃), 3,05 (3H, br s, NCH₃), 3,85-3,90 (9H, d, *J*=8,29 Hz, 3 OCH₃), 4,86 (2H, br s, CH₂-O), 5,11-5,20 (4H, m, 2 CH2), 5,70 (2H, br s, CH₂), 7,16 (2H, s, Hₐᵣₒₘ), 7,25-7,40 (10H, m, Hₐᵣₒₘ)
MS (ES+) : [M+Na]+, m/z : 638

### Exemple 10 ORC036

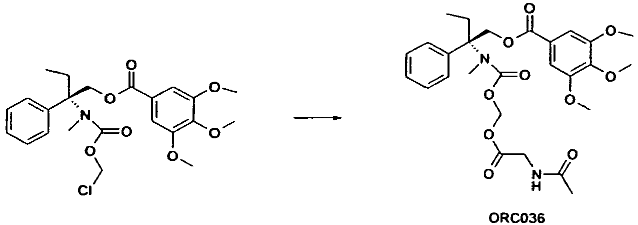

Sous atmosphère d'azote, de la N-acétyl-glycine (0,075 g, 0,00064 mol) est solubilisée dans du DMF (1 mL, 0,01 mol). Du fluorure de césium (0,098 g, 0,00064 mol) est ajouté et le mélange est agité pendant 15 minutes à température ambiante. Le mélange est refroidi à 0°C et le 3,4,5-triméthoxybenzoate de (S) 2-(chlorométhoxycarbonyl méthyl amino)-2-phényl-n-butyle (100,0 mg, 0,0002146 mol) en solution dans le DMF (1 mL) est additionné goutte à goutte. Le mélange réactionnel est chauffé à 70°C pendant 17 heures.

Après refroidissement, de l'acétate d'éthyle (5 mL) est ajouté et la phase organique est lavée avec une solution aqueuse saturée de bicarbonate de sodium, puis une puis une solution aqueuse de chlorure de sodium saturée (2 mL), enfin séchée sur Na2SO4, filtrée et évaporée pour donner le 3,4,5-triméthoxybenzoate de (S)-2-(acetylaminoacétoxyméthoxycarbonyl méthyl amino)-2-phényl-n-butyle sous forme d'une huile jaune (205 mg).
CCM : SiO2, cyclohexane / AcOEt (1/9) Rf 0,34
RMN 1H (CDCl3) : δ (ppm)= 0,86 (3H, t, J=7,34 Hz, CH3-CH2), 2,04 (3H, s, CH3-CO), 2,18-2,26 (1H, m, CH-CH3), 2,32-2,57 (1H, m, CH-CH3), 3,05 (3H, br s, NCH3), 3,86-3,90 (9H, d, J=6,97 Hz, 3 OCH3), 3,96 (2H, br s, CH2), 4,83-4,90 (1H, br d, J=10,54 Hz, CH-O), 5,03-5,06 (1H, br s, CH-O), 5,66-5,74 (2H, br s, CH2), 5,91-5,95 (1H, br s), 7,17 (2H, s, Harom), 7,24-7,37 (5H, m, Harom).
MS (ES+) : [M+Na]+ , m/z : 546

### Exemple 11 ORC037

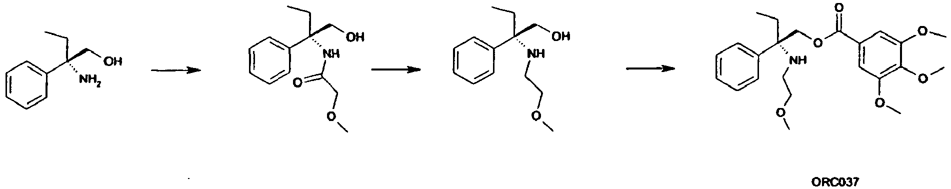

### Etape 1

Sous atmosphère d'azote, (S)-2-amino-2-phényl-butan-1-ol (15,0 g, 0,0908 mol) est solubilisé dans du chlorure de méthylène (150 mL). Du 1-hydroxybenzotriazole (13,5 g, 0,0998 mol), de l'acide méthoxyacétique (7,82 mL, 0,0998 mol) et du N-(3-Diméthylaminopropyl)-N'-éthylcarbodiimide, hydrochloride (19,5 g, 0,0998 mol) sont ajoutés. La solution incolore résultante est agitée à température ambiante pendant 4 jours.

Le mélange est lavé avec de 1' HCl 0,1N (100 mL) et puis une solution saturée de chlorure de sodium (100 mL). La phase organique est séchée sur Na2SO4, filtrée et concentrée pour donner 22,4 g d'huile orange, qui est chromatographiée : SiO2, CH2Cl2 / MeOH (99/1 et 98/2) pour donner 13,12 g de solide légèrement jaune.

RMN ¹H (CDCl₃) δ 0,83 (3H, t, *J*=7,34 Hz, CH₃-CH₂), 1,97-2,18 (2H, m, CH₂-CH₃), 3,49 (3H, s, OCH₃), 3,82-4,10 (4H, m, 2 CH₂), 5,03 (1H, t, J=6,78 Hz, OH), 7,09 (1H, br s, NH), 7,28-7,42 (5H, m, Hₐᵣₒₘ).

### Etape 2

13,120 g, 0,055290 mol de (N-((S)-1-Hydroxyméthyl-1-phényl-propyl)-2-méthoxy-acetamide) sont solubilisés dans le THF (100 mL), sous atmosphère d'azote. La solution refroidie à 10°C, puis du borane-diméthyl sulfide complex (16 mL, 0,16 mol) est additionné goutte à goutte. Le mélange est agité à température ambiante pendant 2 jours. 70 mL, de méthanol sont ajoutés lentement et la solution est agitée 15 minutes. 100 mL d'une solution de carbonate de potassium à 10 %, puis 400mL d'acétate d'éthyle sont ajoutés. La phase organique est lavée avec une solution saturée de chlorure de sodium (100 mL), séchée sur Na2SO4, filtrée puis évaporée pour donner 12,4 g d'huile jaune.

RMN ¹H (CDCl₃) : δ (ppm)= 0,69 (3H, t, *J*=7,34 Hz, CH₃-CH₂), 1,73-1,82 (1H, m), 1,90-2,01 (1H, m), 2,53-2,67 (2H, m, CH₂), 3,30 (3H, s, OCH₃), 3,48 (2H, m, CH2), 3,86-3,98 (2H, dd, *J₁*=10,90 Hz, *J₂*=15,07 Hz), 7,24-7,40 (5H, m, Hₐᵣₒₘ).

### Etape 3

12,40 g (0,05553 mol), de ((S)-2-(2-méthoxy-ethylamino)-2-phényl-butan-1-ol) est introduit dans un ballon muni d'un coude à distillation, puis sont solubilisés dans un mélange Toluène (400 mL) / Ethanol (20 mL).

18,71 g (0,08107 mol) de l'ester méthylique de l'acide 3,4,5-triméthoxy-benzoique, sont ajoutés. La solution est ensuite chauffée à 130°C.

1,5 g, 0,028 mol de méthylate de sodium est ajouté par portions à 130°C. La suspension blanche obtenue est agitée à 130°C pendant 17 heures. De nouveau 1,0 g de méthylate de sodium est ajouté par portions à 130°C et la suspension est agitée pendant 3 heures supplémentaires.

Après refroidissement, le mélange réactionnel est évaporé puis repris avec une solution de NaOH 3N (200 mL).

Le mélange est agité pendant 15 minutes, puis extrait avec de l'acétate d'ethyle (300 mL).

La phase organique est lavée avec une solution saturée de chlorure de sodium (200 mL), séchée sur Na2SO4, filtrée et évaporée pour donner 18,6g de 3,4,5-triméthoxybenzoate de (S)-2-(2-méthoxyéthyl amino)-2-phényl-n-butyle sous forme d'une huile orange.

Le produit est purifié : SiO2, CH2Cl2 puis CH2Cl2 / MeOH (95/5) pour donner 8,3 g d'huile jaune
CCM : SiO2, CH2Cl2 / MeOH (95/5) Rf 0,41
RMN 1H (CDCl3) : δ (ppm)= 0,80 (3H, t, J=7,34 Hz, CH3-CH2), 1,82-1,93 (2H, m), 2,51-2,57 (1H, m), 2,64-2,72 (1H, m), 3,31 (3H, s, OCH3), 3,46-3,51 (2H, m, CH2), 3,85-3,90 (9H, d, J=8,47 Hz, 3 OCH3), 4,51-4,67 (2H, dd, J1 =11,11Hz, J2=24,30 Hz), 7,21-7,53 (7H, m, H arom).
MS (ES+) : [M+H]+ , m/z : 417

Le (S) 2-amino-2-phényl-n-butanol peut être préparé selon la méthode décrite dans les demandes FR 2 765 218 et EP 510 168.

### Exemple 12 ORC050

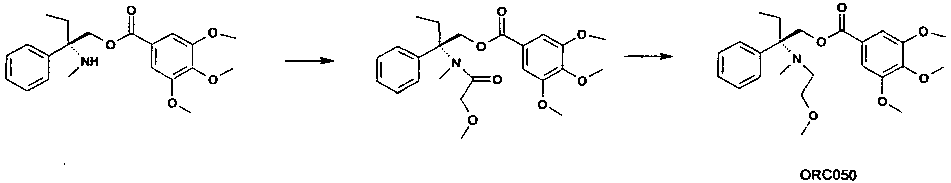

### Etape 1

De l'ester (S)-2-méthylamino-2-phényl-butyl de l'acide 3,4,5-triméthoxy-benzoïque (2,0 g, 0,0054 mol), sous atmosphère d'azote, est solubilisé dans du chlorure de méthylène (20 mL). Du 1-hydroxybenzotriazole (796 mg, 0,00589 mol), de l'acide méthoxyacetique (541 mg, 0,00589 mol) et du N-(3-diméthylaminopropyl)-N'-éthylcarbodiimide hydrochloride (1150 mg, 0,00589 mol) sont ajoutés. La solution incolore est agitée à 40°C pendant 2 jours. Le mélange est lavé avec de l'acide chlorhydrique 0,1N (20 mL), puis avec une solution saturée de chlorure de sodium (20 mL). La phase organique est séchée sur Na2SO4, filtrée et évaporée pour donner 1,84 g d'huile jaune. Le produit est purifié : SiO2, cyclohexane / AcOEt (1/1) pour donner 796 mg d'huile jaune.
CCM : SiO2, cyclohexane / AcOEt (1/1) Rf 0,65
RMN 1H (CDCl3) : δ (ppm)= 0,91 (3H, t, J=7,34 Hz, CH3-CH2), 2,18-2,26 (1H, m), 2,38-2,46 (1H, m), 2,94 (3H, s, NCH3), 3,41 (3H, s, OCH3), 3,85-3,90 (9H, d, J=9,79 Hz, 3 OCH3), 4,09 (2H, br s, CH2), 4,99 (1H, d, J=11,49 Hz), 5,25 (1H, d, J=11,49 Hz), 7,11 (2H, s, Harom), 7,21-7,39 (5H, m, H arom).

### Etape 2

335 mg du produit précédent sont solubilisés dans du THF (1 mL). 0,28 mL (0,0030 mol) de borane-diméthyl sulfide complex est additionné goutte à goutte et le mélange est agité à température ambiante pendant 3 jours. Du méthanol (0,7 mL) est ajouté au mélange réactionnel et la solution est agitée pendant 30 minutes. Une solution à 10 % de carbonate de potassium (1 mL) et puis 3 mL d'acétate d'éthyle (3 mL) sont ajoutés. La phase organique est lavée avec une solution saturée de chlorure de sodium (1 mL), séchée sur Na2SO4, filtrée et évaporée pour donner 135 mg d'huile légèrement jaune. Le produit est purifié : SiO2, cyclohexane / AcOEt (7/3) pour donner 221 mg de 3,4,5-triméthoxybenzoate de (S)-2-(2-méthoxyéthyl méthyl amino)-2-phényl-n-butyle sous forme d'une huile incolore.

RMN ¹H (CDCl₃) : δ (ppm)= 0,68 (3H, t, *J*=7,34 Hz, CH₃-CH₂), 1,87 (2H, q, *J*=7,35 Hz, CH₂-CH₃), 2,44 (3H, s, NCH₃), 2,84 (2H, t, *J*=6,59 Hz, CH₂), 3,28 (3H, s, OCH₃), 3,44 (2H, t, J=6,59 Hz), 3,82-3,90 (9H, d, *J*=16,58 Hz, 3 OCH₃), 4,75-4,84 (2H, dd, *J₁*=3,77 Hz, *J₂*=11,87 Hz, CH₂), 7,21-7,50 (7H, m, Hₐᵣₒₘ).
MS (ES+) : [M+H]+ , m/z : 431

### Exemple 13 ORC051

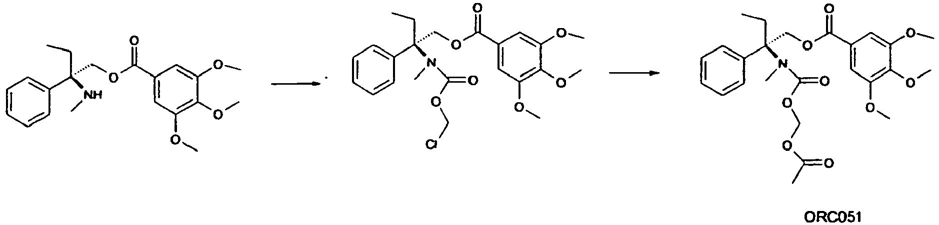

### Etape 1

Sous atmosphère d'azote, 200,0 mg (0,0005356 mol) de 3,4,5-triméthoxybenzoate de (R) 2-(chlorométhoxycarbonyl méthyl amino)-2-phényl-n-butyle sont solubilisés dans du chlorure de méthylène (1,0 mL). La solution incolore est refroidie à -1°C et du chlorométhyl chloroformate (210 mg, 0,0016 mol) est ajouté goutte à goutte. Le mélange réactionnel est agité à température ambiante pendant 17 heures. La solution incolore est ensuite refroidie à - 5°C et du chlorométhyl chloroformate (0,106 mg, 1,5 éq) est additionné goutte à goutte, à nouveau. La solution est agitée à température ambiante pendant 3 heures. Le mélange est refroidi à 0°C et du chlorométhyl chloroformate (106 mg, 1,5 éq) est additionné goutte à goutte, à nouveau. La solution est agitée à température ambiante pendant 2 heures, puis chauffée à 40°C pendant 1 heure, puis est agité à température ambiante pendant 15 heures..

La phase organique est traitée par une solution saturée de bicarbonate (3,0 mL), après avoir rajouté 3 mL de chlorure de méthylène. La phase organique est lavée avec une solution saturée de chlorure de sodium (3,0 mL), séchée sur Na2SO4, filtrée puis évaporée pour donner 150 mg d'une huile légèrement jaune.

RMN ¹H (CDCl₃) : δ (ppm)= 0,90 (3H, t, *J*=7,34 Hz, CH₃-CH₂), 2,17-2,24 (1H, m, CH-CH₃), 2,36-2,46 (1H, m, CH-CH₃), 3,01 (3H, br s, NCH₃), 3,87-3,89 (9H, d, *J*=5,84 Hz, 3 OCH₃), 4,93-4,97 (2H, br d, *J*=10,55 Hz, CH₂-O), 5,69 (2H, br s, CH₂-Cl), 7,17 (2H, s, H ₐᵣₒₘ), 7,25-7,36 (5H, m, H ₐᵣₒₘ).

### Etape 2 :

Sous atmosphère d'azote, l'acide 3,4,5-triméthoxy-benzoïque (R)-2-(chlorométhoxy-carbonyl-méthyl-amino)-2-phényl-butyl ester (150,0 mg, 0,0003219 mol) est solubilisé dans du DMF (2,0 mL). La solution est refroidie à 0°C et de l'acétate de sodium (195 mg, 0,000965 mol) solubilisé dans du DMF (2,0 mL) est additionné goutte à goutte. Le mélange est chauffé à 70°C pendant 15 heures. Après refroidissement, l'acétate d'éthyle (13,0 mL) et une solution saturée de bicarbonate (13,0 mL) sont ajoutés.

Le mélange est agité 15 minutes à température ambiante. La phase organique est lavée avec est lavée avec une solution saturée de chlorure de sodium (10 mL), séchée sur NaSO4, filtrée puis évaporée pour donner une huile jaune. Le produit obtenu est purifié sur colonne: SiO2, Cyclohexane/AcOEt (8/2) pour donner, après évaporation, 116 mg de 3,4,5-triméthoxybenzoate de (R)-2-(acétoxyméthoxycarbonyl méthyl amino)-2-phényl-n-butyle sous forme d'une huile jaune.
CCM : SiO2, Cyclohexane/AcOEt (1/1) Rf: 0,64
RMN 1H (CDCl3) : δ (ppm)= 0,87 (3H, t, J=7,34 Hz, CH3-CH2), 1,99 (3H, br s, CH3-CO), 2,14-2,23 (1H, m, CH-CH3), 2,40-2,47 (1H, m, CH-CH3), 3,01 (3H, br s, NCH3), 3,87-3,89 (9H, d, J=5,65 Hz, 3 OCH3), 4,87-4,92 (2H, br d, J=10,73 Hz, CH2-O), 5,65 (2H, br s, CH2-Cl), 7,17 (2H, s, Harom), 7,25-7,36 (5H, m, Harom).
MS (ES+) : [M+NH4]+ , m/z : 507

### Exemple 14 ORC 052

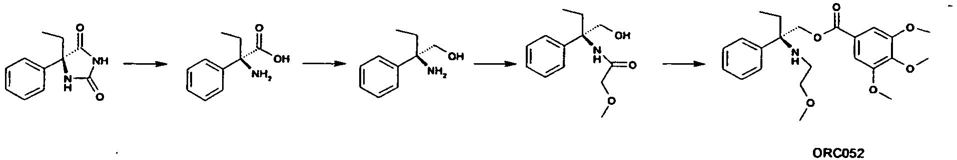

### Etape 1 :

Dans un autoclave, de l'hydroxyde de sodium (17,120 g, 0,42803 mol) est solubilisé dans l'eau (74 mL, 4,1 mol), puis la (5R)-5-éthyl-5-phénylimidazolidine-2,4-dione (20,0 g, 0,0979 mol) est additionnée par portions. Le mélange réactionnel est chauffé à 130°C sous agitation pendant 48 heures. Le mélange est refroidi et un précipité blanc se forme.

Le pH est ramené à 7 puis le précipité est lavé à l'acétate d'éthyle. Le mélange est dilué dans 300mL d'eau refroidie à 0°C. Le pH est ramené à 1 avec de l'HCl 12 M (50,0 mL). Le précipité est entièrement solublilisé. Tout en maintenant le mélange à 0°C, le pH est ramené à 7 avec 20 mL d'une solution de NaOH 5N afin d'obtenir un précipité blanc. Le mélange est filtré, le précipité est lavé avec de l'eau (150 mL) puis séché sous vide à 40°C pour donner 22,8 g d'un solide blanc.

RMN ¹H (MeOD) : δ (ppm)= 1,06 (3H, t, *J*=7,34 Hz, CH₃-CH₂), 2,30-2,38 (2H, m, CH₂-CH₃), 7,33-7,46 (3H, m, H ₐᵣₒₘ), 7,55-7,59 (2H, m, H ₐᵣₒₘ).

### Etape 2 :

Sous atmosphère d'azote, le (2R)-2-amino-2-phénylbutanoic acid (21,60 g, 0,1205 mol) est solubilisé dans le THF (200 mL). Du borane-THF 1M en solution dans le THF (94 mL, 0,96 mol) est ajouté goutte à goutte.

Le mélange est chauffé à 70°C sous agitation durant 48 heures. Du borane-THF 1M en solution dans le THF (47 mL, 4 eq) est ajouté goutte à goutte. Le mélange est chauffé à 70°C pendant 2 heures. L'opération est renouvelée.

Le mélange est finalement refroidi vers 0°C puis une solution saturée de bicarbonate de sodium (100,0 mL) est ajoutée. Après ajout de chlorure de méthylène, la phase organique est lavée par une solution saturée de chlorure de sodium, enfin séchée sur Na2SO4, puis filtrée et évaporée pour donner 13,06 g d'un solide légèrement jaune.

RMN ¹H (MeOD) : δ (ppm)= 0,59 (3H, t, *J*=7,34 Hz, CH₃-CH₂), 1,57-1,69 (1H, m, CH-CH₃), 1,71-1,84 (1H, m, CH-CH₃), 3,52-3,61 (2H, dd, *J*₁=10,92 Hz, *J*₂=4,14 Hz, CH₂-OH), 7,09-7,33 (5H, m, Hₐᵣₒₘ).

### Etape 3 :

Sous atmosphère d'azote, le (R)-2-amino-2-phényl-butano-lol (13,060 g, 0,079040 mol) est solubilisé dans du chlorure de méthylène (300 mL). De l'acide méthoxy acétique (7,99 g, 0,0869 mol), du 1-hydroxybenzotriazole (11,8 g, 0,0870 mol) et du N-(3-diméthylaminopropyl)-N'-éthylcarbodiimide, (HCl) (16,70 g, 0,08537 mol) sont ajoutés directement dans le mélange avec un minimum de chlorure de méthylène. La solution est agitée à température ambiante pendant 17 heures. Le mélange est lavé avec de l'HCl 0,1 M (300,0 mL). La phase aqueuse est extraite avec du chlorure de méthylène (3 fois 200,0 mL).

Les phases organiques sont regroupées, lavées avec une solution de chlorure de sodium saturée (600,0 mL), séchées sur Na2SO4, filtrées, puis évaporées pour donner 14 g d'une huile jaune. Le produit est purifié: SiO2, CH2Cl2 / MeOH (99/1, 98/2, 100 % MeOH) pour donner 5,17 g d'une huile légèrement jaune.

RMN ¹H (CDCl₃) : δ (ppm)= 0,83 (3H, t, *J=7,34* Hz, CH₃-CH₂), 1,96-2,16 (2H, m, CH₂-CH₃), 3,49 (3H, s, OCH₃), 3,82-4,10 (4H, m, 2 CH₂), 5,03 (1H, t, *J=6,78* Hz, OH), 7,09 (1H, br s, NH), 7,26-7,42 (5H, m, H ₐᵣₒₘ).

### Etape 4 :

Sous atmosphère d'azote, le N-(1-hydroxyméthyl-1-phényl-propyl)-2-méthoxy-acetamide (5,170 g, 0,02179 mol) est solubilisé dans du THF (50,0 mL,). Le mélange est refroidi à 10°C puis du borane-diméthyl sulfide complex (6,2 mL, 0,065 mol) est ajouté goutte à goutte. Le mélange est agité à température ambiante pendant 24 heures. Le mélange est refroidi à 10°C puis du borane-diméthyl sulfide complex (6,2 mL, 3 éq) est ajouté goutte à goutte. Le mélange est agité à température ambiante pendant 3 heures. Le mélange est ensuite refroidi à 0°C et du méthanol (27,0 mL) est additionné goutte à goutte. 40mL d'une solution à 10 % de carbonate de potassium, puis 80mL d'acétate d'éthyle sont ajoutés au mélange. La phase aqueuse est extraite avec de l'acétate d'éthyle. La phase organique est lavée avec une solution saturée de chlorure de sodium, séchée sur Na2SO4, filtrée puis évaporée pour donner 4,92 g d'une huile jaune.

RMN ¹H (CDCl₃) : δ (ppm)= 0,66 (3H, t, *J*=7,34 Hz, CH₃-CH₂), 1,53-1,69 (1H, m), 1,73-1,89 (1H, m), 2,40-2,60 (2H, m, CH₂), 3,36 (3H, s, OCH₃), 3,43-3,48 (2H, m, CH₂), 3,72-3,88 (2H, dd, *J*₁=10,90 Hz, *J*₁=15,07 Hz), 7,21-7,40 (5H, m, Hₐᵣₒₘ).

### Etape 5 :

Dans un ballon muni d'un coude de distillation, le (R)-2-(2-méthoxy-ethylamino)-2-phényl-butan-1-ol (4,840 g, 0,02167 mol) est solubilisé dans un mélange Toluène (150 mL) /méthanol (7,60 mL). L'ester méthylique de l'acide 3,4,5-triméthoxy-benzoïque, (5,50 g, 0,0238 mol) est ajouté et le mélange est chauffé à 130°C. Du méthylate de sodium (0,58 g, 0,011 mol) est additionné par portions et le mélange est agité à 130°C pendant 3 heures.

Du méthylate de sodium (0,47 g, 0,0087 mol) est à nouveau ajouté par portions et le mélange est agité à 130°C pendant 15 heures. Le mélange est refroidi puis évaporé. 70mL d'une solution aqueuse 3M d'hydroxyde de sodium sont ajoutés puis le mélange est agité pendant 15 minutes avant d'être extrait avec de l'acétate d'éthyle. La phase organique est lavée avec une solution saturée de chlorure de sodium, séchée sur Na2SO4, filtrée, puis évaporée pour donner 8,87 g d'une huile orange. Le produit est purifié sur colonne : SiO2, Cyclohexane / AcOEt (9/1, 8/2) puis 100 % AcOEt) pour donner 4,37 g de 3,4,5-triméthoxybenzoate de (R)-2-(2-méthoxyéthyl amino)-2-phényl-n-butyle sous forme d'une huile légèrement jaune.
CCM: SiO2 CH2Cl2 / MeOH (95/5) ; Rf: 0,34
RMN 1H (CDCl3), δ (ppm)= 0,80 (3H, t, J=7,34 Hz, CH3-CH2), 1,79-1,95 (2H, m), 2,50-2,57 (1H, m), 2,64-2,72 (1H, m), 3,31 (3H, s, OCH3), 3,46-3,51 (2H, m, CH2), 3,86-3,89 (9H, d, J=8,28 Hz, 3 OCH3), 4,51-4,67 (2H, dd, J1=11,11Hz, J2=24,30 Hz), 7,21-7,53 (7H, m, H arom).
MS (ES+) : [M+H]+ , m/z : 417.

### Exemple 15 ORC055

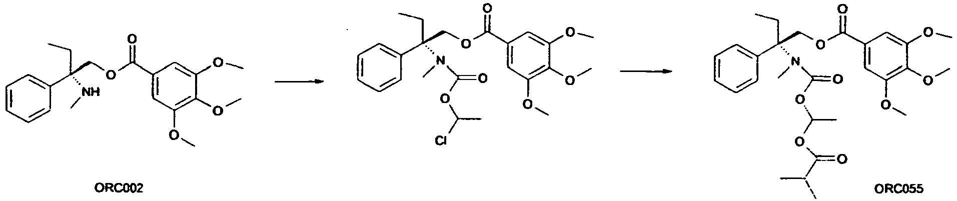

### Etape 1 :

Sous azote, 500 mg (1,34 mmol; 1,0 éq.) de 3,4,5-triméthoxybenzoate de (S) 2-méthylamino-2-phényl-n-butyle sont dissous dans 1 ml de dichlorométhane. Le mélange est refroidi à 2°C. A cette température, 440 µl (4,0 mmol; 3,0 éq.) de α-chloroéthyl chloroformate sont additionnés goutte à goutte. Le mélange est agité à température ambiante pendant 5 heures. La réaction est suivie par CCM. Le dichlorométhane est évaporé à l'azote. Le résidu obtenu sous forme d'une huile incolore est utilisé dans l'étape suivante sans purification supplémentaire.

¹H-RMN (CDCl₃, 300MHz) : δ (ppm)= 0,94 (m, 3H, CH₃); 1,59 (m, 3H, CH₃); 2,22 (m, 1H, CH₂ diastéréotope); 2,45 (m, 1H, CH₂ diastéréotope); 3,08 (br, 3H, NCH₃); 3,90 (s, 3H, OCH₃); 3,91 (s, 3H, OCH₃); 3,92 (s, 3H, OCH₃); 5.00 (br, 2H, OCH₂); 6.48 (br, 1H, CICH); 7,19-7,39 (m, 7H, ArH).
R_{f}(SiO₂, Cyclohexane/Acétate d'éthyle, 6/ 4): 0,8

### Etape 2 :

Le 3,4,5-trimethoxy-benzoate de (S) 2-[(1-chloro-éthoxycarbonyl)-méthyl-amino]-2-phenyl-n-butyl obtenu à l'étape précédente est dissous dans 2 mL de N,N-diméthylformamide. 807 mg (3,67 mmol, 2,7 éq.) d'isobutyrate de césium sont ajoutés. Le mélange est agité à 55°C pendant une nuit. La réaction est suivie par CCM.

10 ml d'eau sont ajoutés au mélange. Le produit est extrait à l'acétate d'éthyle (3 fois 10 mL) puis la phase organique est lavée successivement avec de l'eau et une solution de chlorure de sodium saturée (15 mL). Les phases organiques sont séchées sur Na₂SO₄, filtrées puis concentrées à sec pour restituer une huile marron. L'huile est purifiée par chromatographie flash sur gel de silice (éluant : cyclohexane/acétate d'éthyle : 85/15) pour donner 105 mg du produit attendu 3,4,5-triméthoxy-benzoate de (S) 2-[(1-isobutyryloxy-éthoxycarbonyl)-méthyl-amino]-2-phényl-n-butyl sous la forme d'une huile incolore (rendement = 15 %).

1H-RMN (CDCl3, 300MHz) : δ (ppm)= 0,91 (m, 3H, CH3 éthyl); 1,10 (m, 6H, 2 x CH3 isopropyl); 2,2 (br, 2H, CH2 éthyl); 2,4 (br, 3H, CH3CH); 3,05 (br, 4H, CH3N + CH isopropyl); 3,89 (s, 6H,CH3O); 3,89 (s, 6H,CH3O); 3,91 (s, 3H, CH3O) ; 4,89-4.98 (br, 2H, OCH2); 6,71 (br, 1H, OCHO); 7,17-7,39 (m, 7H, ArH).
Rf (Si02, cyclohexane/acétate d'éthyle, 6/4) : 0,9
MS (ES+) : [M+NH4]+, m/z : 549,2

### Exemple 16 ORC056

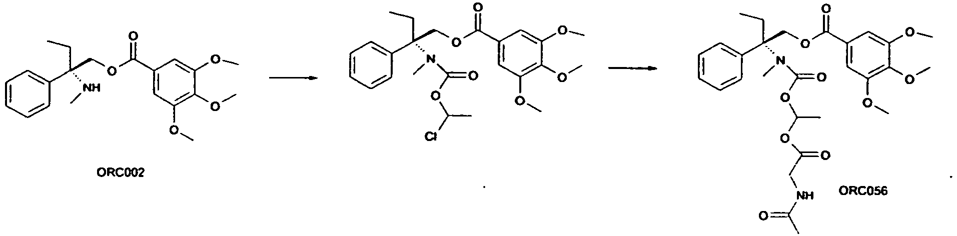

### Etape 1 :

Sous azote, 2 g (5,4 mmol; 1,0 éq.) de 3,4,5-triméthoxybenzoate de (S) 2-méthylamino-2-phényl-n-butyle sont dissous dans 4 ml de dichlorométhane. Le mélange est refroidi à 2 °C.

A cette température, 884 µl (8,0 mmol; 1,5 éq.) de α-chloroethyl chloroformate sont additionnés goutte à goutte. Le mélange est agité à température ambiante pendant 5 heures. La réaction est suivie par CCM. Le dichlorométhane est évaporé à l'azote. Le résidu obtenu sous forme d'une huile incolore est utilisé dans l'étape suivante sans purification supplémentaire.
¹H-RMN (CDCl₃, 300MHz) : δ (ppm)= 0,94 (m, 3H, CH₃); 1,59 (m, 3H, CH₃); 2,22 (m, 1H, CH₂ diastéréotope); 2,45 (m, 1H, CH₂ diastéréotope); 3,08 (br, 3H, NCH₃); 3,90 (s, 3H, OCH₃); 3,91 (s, 3H, OCH₃); 3,92 (s, 3H, OCH₃); 5,00 (br, 2H, OCH₂); 6,48 (br, 1H, ClCH); 7,19-7,39 (m, 7H, ArH).
R_{f} (SiO₂, Cyclohexane/Acétate d'éthyle, 6/ 4) : 0,8

### Etape 2 :

Le 3,4,5-trimethoxy-benzoate de (S) 2-[(1-chloro-ethoxycarbonyl)-méthyl-arnino]-2-phenyl-n-butyl obtenu à l'étape précédente est dissous dans 3 mL de N,N-diméthylformamide. 2,0 g (8,03 mmol, 1,5 éq.) d'(acétylamino)acétate de césium sont ajoutés. Le mélange est agité à 55°C pendant une nuit. La réaction est suivie par CCM.

20 ml d'eau sont ajoutés au mélange. Le produit est extrait à l'acétate d'éthyle (3 fois 50 mL) puis la phase organique est lavée successivement avec de l'eau et une solution de chlorure de sodium saturée (10 mL). Les phases organiques sont séchées sur Na₂SO₄, filtrées puis concentrées à sec pour restituer une huile marron. L'huile est purifiée par chromatographie flash sur gel de silice (éluant : cyclohexane/ Acétate d'éthyle : 3/7) pour donner 200 mg du produit attendu 3,4,5-trimethoxy-benzoate de (S) 2-{[1-(2-acetylamino-acetoxy)-éthoxycarbonyl]-méthyl-amino}-2-phényl-n-butyl sous la forme d'un solide brun (rendement = 7 %).

1H-RMN (CDCl3, 300MHz) : δ (ppm)= 0,81 (m, 3H, CH3 éthyl); 1,94 (s, 3H, CH3CO); 2,15 (m, 2H, CH2 éthyl); 2,35 (m, 3H, CH3CH); 3,0 (br, 3H, CH3N); 3,90 (m, 9H, OCH3); 4,80 (m, 2H, OCH2); 5,95 (br, 2H, CH2N); 6,65 (br, 1H, OCHO); 7,10-7,30 (m, 7H, ArH).
Rf (Si02, Cyclohexane/Acétate d'éthyle, 3/7) : 0.2
[M+NH4]+, m/z : 578,2

Les produits de formule générale (I) peuvent être administrés par voie orale, parentérale, perlinguale, rectale, en aérosols ou sous forme topique.

La présente invention concerne également les compositions pharmaceutiques comprenant au moins un ester dérivé 2-amino-2-phényl-alkanol de formule générale (I) et/ou leurs sels, lorsqu'ils existent, à l'état pur ou sous forme d'association avec un ou plusieurs diluants ou adjuvants compatibles et pharmaceutiquement acceptables.

Ces compositions peuvent être présentées sous forme de compositions solides, notamment sous forme de comprimés, de comprimés enrobés, de pilules, de gélules, de poudres à mettre en solution ou en suspension, ou de granulés, ou sous forme de compositions liquides comme des solutions ou des suspensions injectables, des solutions ou des suspensions buvables, des sirops, des émulsions, des élixirs contenant des diluants inertes tels que l'eau ou l'huile de paraffine ou sous forme de suppositoires, de crèmes, de pommades et de lotions ou encore sous forme de compositions à pulvériser. Ces formes pharmaceutiques sont préparées selon les méthodes usuelles.

Dans les compositions solides pour administration orale le produit actif selon l'invention est mélangé à un ou plusieurs diluants ou adjuvants inertes, tels que par exemple le saccharose, le lactose, l'amidon ou ses dérivés, la cellulose microcristalline, la silice colloïdale, la povidone, le talc, la gomme arabique.

Ces compositions peuvent comprendre des substances autres que les diluants, par exemple un lubrifiant tel que le stéarate de magnésium ou un enrobage destiné à une libération contrôlée.

Les compositions liquides pour administration orale, peuvent comprennent des véhicules aqueux ou non comme des diluants et peuvent aussi comprendre d'autres substances comme par exemple des produits mouillants, édulcorants ou aromatisants. Les compositions non aqueuses peuvent comprendre des corps gras d'origine animale ou végétale, des dérivés paraffiniques, des glycols, de la lécithine de soja.

Les compositions administrables par voie parentérale, sont plus particulièrement des compositions administrables par voie intramusculaire ou intra-veineuse. Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants, stabilisants, et/ou des conservateurs.

La stérilisation peut se faire de plusieurs façons, par exemple à l'aide d'un filtre bactériologique, par irradiation ou par chauffage. Les compositions peuvent également être préparées sous forme de compositions solides stériles qui sont dissoutes au moment de l'emploi dans de l'eau stérile ou tout autre milieu stérile injectable.

Les compositions par administration rectale sont des suppositoires ou des capsules rectales, qui contiennent outre le principe actif, des excipients tels que le beurre de cacao, des glycérides semi- synthétiques ou des polyéthylèneglycols.

Les compositions pour administration topique peuvent être par exemple des patches qui contiennent outre le principe actif des excipients compatibles tels que l'huile de silicone, de la paraffine.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 pm, par exemple le dextrane, le mannitol ou le lactose.

En thérapeutique humaine, le médecin déterminera la posologie qu'il estime la plus appropriée en fonction du traitement, en fonction de l'âge, du poids, et des autres facteurs propres au sujet à traiter. La dose usuelle, variable selon le sujet traité et l'affection en cause, peut être, par exemple, de 50 mg à 2 g par jour pour un adulte, par voie orale.

L'exemple suivant illustre une composition selon l'invention.

### Exemple

On prépare une formulation administrable par voie orale et ayant la composition suivante :
3,4,5-triméthoxybenzoate de (S) 2-(chlorométhoxycarbonyl méthyl amino)-2-phényl-n-butyle 100 mg
lactose monohydrate,
amidon de maïs modifié;
hydroxypropyl méthylcellulose,
carboxyméthylamidon de sodium,
acide tartrique,
silice colloïdale,
stéarate de magnésium,
macrogol 4000,
dioxyde de titane.

## Revendications

1. Un ester dérivé de 2-amino-2-phényl-alkanol de formule générale : 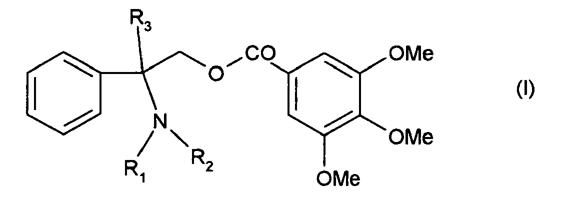 dans laquelle :
R₁ est un atome d'hydrogène, un radical alkyle contenant 1 à 4 atomes de carbone en chaîne droite ou ramifiée, un radical alkyle contenant 2 à 4C en chaîne droite ou ramifiée substitué par hydroxy, alkoxy, alkylthio, acyloxy, amino, alkylamino, dialkylamino, alkylcarbamoyloxy, alkoxycarbonylamino, uréido ou alkyluréido,
R₂ est un radical -CO-R dans lequel R est un radical alkyle, un radical aryle, hétérocyclyle, benzyle ou hétérocyclylméthyle,
un radical -CO-Y-R₄ pour lequel Y est un hétéroatome choisi parmi -O-, -S-, -NH-, -Nalk-pour lequel alk est un radical alkyle droit ou ramifié contenant 1 à 4C, et R₄ est choisi parmi les radicaux alkyle, aryle, aralkyle ou hétérocyclylalkyle, pouvant être substitués par un ou plusieurs atomes d'halogène ou radicaux hydroxy, alkyle contenant de 1 à 4C en chaîne droite ou ramifiée, alkoxy, alkylthio, acylaminoalkylthio, alkoxycarbonyle ou acylamino dont les restes alkyle contiennent 1 à 4C en chaîne droite ou ramifiée, ou oxo, ou pouvant être substitués par un radical R₅COO- dans lequel R₅ représente un radical alkyle éventuellement substitué par benzyloxycarbonylamino, acylamino ou par le reste d'un aminoacide, ou représente un radical hétérocyclyle, ou bien
R₂ est un radical alkyle contenant 2 à 4C substitué par hydroxy, alkoxy, alkylthio, acyloxy, amino, alkylamino, dialkylamino dont les parties alcoyle peuvent former avec l'atome d'azote auquel elles sont attachées, un hétérocycle à 5 ou 6 chaînons portant éventuellement un autre hétéroatome choisi parmi l'oxygène ou l'azote, ou substitué par alkylcarbamoyloxy, alkoxycarbonylamino, uréido ou alkyluréido, étant entendu que ledit radical alkyle substitué est en chaîne droite ou ramifiée et comporte au moins 2 atomes de carbone entre l'atome d'azote portant R₂ et le substituant ;
R₃ est un radical alkyle contenant 1 à 4 atomes de carbone en chaîne droite ou ramifiée,
sous ses formes R ou S ou leurs mélanges, ainsi que ses sels pharmaceutiquement acceptables, lorsqu'ils existent,.

2. Un ester dérivé de 2-amino-2-phényl-alkanol selon la revendication 1, **caractérisé en ce qu'**il répond à la formule générale (I) pour laquelle R₁ et R₃ sont définis comme dans la revendication 1, et R₂ dans -NR₁R₂ est un radical -CO-O-R₄ pour lequel R4 a une structure :
C(alk)-O-CO-R₅ (X)
alk étant un radical alcoyle contenant 1 à 6 atomes de carbone en chaîne droite ou ramifiée et R₅ est défini comme dans la revendication 1, ledit ester étant désigné (Ia), ou bien **caractérisé en ce qu'**il répond à la formule générale (I) pour laquelle R₃ est défini comme dans la revendication 1, et dans -NR₁R₂, R₁ est un atome d'hydrogène et R₂ est un radical -CO-R tel que défini dans la revendication 1, ledit ester étant désigné (Ia').

3. Un ester dérivé de 2-amino-2-phényl-alkanol selon la revendication 2, désigné (Ia), **caractérisé en ce qu'**il répond à la formule générale (I) pour laquelle R₁ est un atome d'hydrogène, un radical alkyle contenant 1 à 4 atomes de carbone, de préférence méthyle, ou un radical 2-méthoxyéthyle et R₂ et R₃ sont définis comme dans la revendication 2, pour l'ester (Ia).

4. Un ester dérivé de 2-amino-2-phényl-alkanol selon la revendication 1, **caractérisé en ce qu'**il répond à la formule générale (I) pour laquelle R₁ et R₃ sont définis comme dans la revendication 1 et R₂ dans -NR₁R₂ est un radical alkyle substitué tel que défini pour R₂ dans la revendication 1, ledit ester étant désigné (Ib).

5. Un procédé de préparation d'un dérivé selon la revendication 1, **caractérisé en ce que** l'on fait agir un dérivé de formule générale : 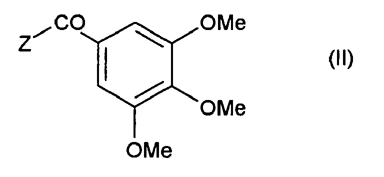 dans laquelle Z est un atome d'halogène, un radical hydroxy ou le reste d'un ester réactif, sur le dérivé de 2-amino-2-phényl alkanol, de formule générale : 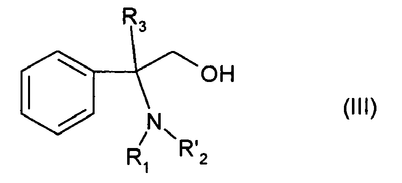 dans laquelle R₁ et R₃ sont définis comme précédemment et R'₂ est un atome d'hydrogène ou est défini comme R₂ dans la revendication 1, puis le cas échéant, lorsque l'un de R'₂ ou R₁ est l'atome d'hydrogène, effectue la substitution de l'amine de l'ester dérivé de 2-amino-2-phényl-alkanol obtenu, de formule générale : 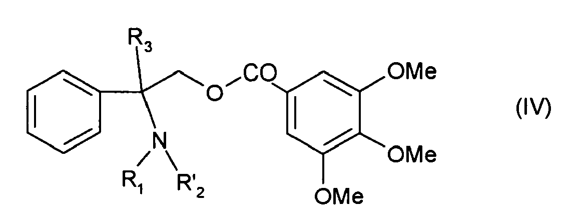 dans laquelle R₁, R'₂ et R₃ sont définis comme ci-dessus,
• soit, lorsque R'₂ est H, et si l'on veut obtenir des dérivés pour lesquels R₂ est -CO-R, par action d'un dérivé réactif de l'acide de formule générale
R-COOH (V)
dans laquelle R est défini comme dans la revendication 1,
• soit, lorsque R'₂ est H, et si l'on veut obtenir des dérivés pour lesquels R₂ est -CO-Y-R₄, Y étant O, S, NH ou Nalk
- ou bien, par action du phosgène, suivie de la réaction avec l'alcool, le thiol ou l'amine de formule générale :
R₄-YH (VI)
.dans laquelle R₄ est un radical alkyle éventuellement substitué et dont le cas échéant les fonctions altérables dans la réaction sont préalablement protégées, ou un radical aryle, aralkyle ou hétérocyclylalkyle, et Y est l'atome d'oxygène ou de soufre, ou un radical NH ou Nalk
- ou bien, par action de l'halogénure de formule générale :
R₄-Y-COHal (VII)
dans laquelle R₄ est défini comme dans la revendication 1, de préférence aryle ou alkyle ramifié, Y est l'atome d'oxygène ou de soufre et Hal est un atome d'halogène, de préférence le chlore,
- ou bien, lorsque l'on veut obtenir un radical R₄ portant la substitution -C(alk)-O-CO-R₅ pour laquelle alk est défini comme dans la revendication 2 et R₅ est défini comme dans la revendication 1, par action du chloroalkylchloroformate, suivie de la réaction du produit obtenu avec un sel alcalin de l'acide correspondant R₅COOH, ou encore le sel d'argent ou un sel d'ammonium quaternaire de cet acide.
• soit, lorsque R'₂ est H, et si l'on veut obtenir des dérivés pour lesquels R₂ est alkyle substitué, ou bien lorsque l'on a obtenu un dérivé de formule générale (IV) pour lequel R₁ est un atome d'hydrogène et R'₂ est défini comme R₂ dans la revendication 1, et si l'on veut obtenir un produit de formule générale (I) pour lequel R₁ est alkyle éventuellement substitué, par acylation par un halogénure d'acide ou un ester réactif de structure :
R₂-CO-Z (VIIIa)
ou
R₁-CO-Z (VIIIb)
dans laquelle R₁ ou R₂ sont définis comme ci-dessus et Z est un atome d'halogène ou le reste d'un ester réactif, suivie de la réduction de l'amide formé en une amine.
• soit encore, lorsque l'on a obtenu un dérivé de formule générale (IV) pour lequel R₁ est un atome d'hydrogène et R'₂ est défini comme R₂ dans la revendication 1, et si l'on veut obtenir un produit de formule générale (I) pour lequel R₁ est alkyle, par action d'un dérivé halogéné de formule
R₁-X (IX)
dans laquelle R₁ est un radical alkyle et X est un atome d'halogène ou un radical sulfonique, en présence d'une base,
puis transforme éventuellement le produit obtenu en un sel pharmaceutiquement acceptable, lorsqu'ils existent.

6. Composition pharmaceutique comprenant au moins un produit selon la revendication 1, à l'état pur ou sous forme d'association avec un ou plusieurs diluants ou adjuvants compatibles et pharmaceutiquement acceptables.

## Claims

1. A 2 -amino-2-phenyl-alkanol ester derivative of general formula: 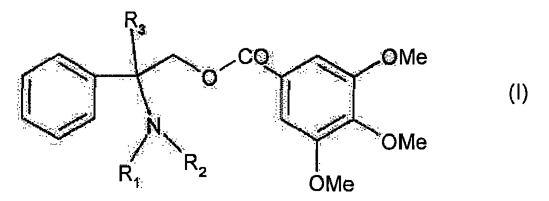 in which:
R₁ is a hydrogen atom, an alkyl radical containing 1 to 4 carbon atoms in a straight or branched chain, an alkyl radical containing 2 to 4C in a straight or branched chain substituted by hydroxy, alkoxy, alkylthio, acyloxy, amino, alkylamino, dialkylamino, alkylcarbamoyloxy, alkoxycarbonylamino, ureido or alkylureido,
R₂ is a -CO-R radical in which R is an alkyl radical, an aryl, heterocyclyl, benzyl or heterocyclylmethyl radical,
a -CO-Y-R₄ radical for which Y is a heteroatom chosen from -O-, -S-, -NH-, -Nalk- for which alk is a straight or branched alkyl radical containing 1 to 4C, and R₄ is chosen from the alkyl, aryl, aralkyl or heterocyclylalkyl radicals, that could be substituted by one or more halogen atoms or hydroxy, alkyl containing from 1 to 4C in a straight or branched chain, alkoxy, alkylthio, acylaminoalkylthio, alkoxycarbonyl or acylamino radicals the alkyl residues of which contain 1 to 4C in a straight or branched chain, or oxo, or capable of being substituted by an R₅COO- radical in which R₅ represents an alkyl radical optionally substituted by benzyloxycarbonylamino, acylamino or by an amino acid residue, or represents a heterocyclyl radical, or
R₂ is an alkyl radical containing 2 to 4C substituted by hydroxy, alkoxy, alkylthio, acyloxy, amino, alkylamino, dialkylamino the alkyl residues of which can form, with the nitrogen atom to which they are attached, a heterocycle having 5 or 6 members, optionally bearing another heteroatom chosen from oxygen or nitrogen, or substituted by allcylcarbamoyloxy, alkoxycarbonylamino, ureido or alkylureido, it being understood that said substituted alkyl radical is in a straight or branched chain and comprises at least 2 carbon atoms between the nitrogen atom bearing R₂ and the substituent;
R₃ is an alkyl radical containing 1 to 4 carbon atoms in a straight or branched chain,
in its R or S forms or their mixtures, as well as its pharmaceutically acceptable salts, when these exist.

2. A 2-amino-2-phenyl-alkanol ester derivative according to claim 1, **characterized in that** it corresponds to general formula (I) for which R₁ and R₃ are defined as in claim 1, and R₂ in -NR₁R₂ is a -CO-O-R₄ radical for which R₄ has a structure:
C(alk)-O-CO-R₅ (X)
alk being an alkyl radical containing 1 to 6 carbon atoms in a straight or branched chain and R₅ is defined as in claim 1, said ester being called (Ia), or **characterized in that** it corresponds to general formula (I) for which R₃ is defined as in claim 1, and in -NR₁R₂, R₁ is a hydrogen atom and R₂ is a -CO-R radical as defined in claim 1, said ester being called (Ia').

3. A 2-amino-2-phenyl-alkanol ester derivative according to claim 2, called (Ia), **characterized in that** it corresponds to general formula (I) for which R₁ is a hydrogen atom, an alkyl radical containing 1 to 4 carbon atoms, preferably methyl, or a 2-methoxyethyl radical and R₂ and R₃ are defined as in claim 2, for the ester (Ia).

4. A 2-amino-2-phenyl-alkanol ester derivative according to claim 1, **characterized in that** it corresponds to general formula (I) for which R₁ and R₃ are defined as in claim 1, and R₂ in -NR₁R₂ is a substituted alkyl radical as defined for R₂ in claim 1, said ester being called (Ib).

5. A method for the preparation of a derivative according to claim 1, **characterized in that** a derivative of general formula: 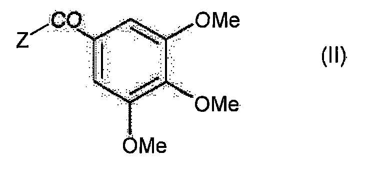 in which Z is a halogen atom, a hydroxy radical or the residue of a reactive ester, is reacted with the 2-amino-2-phenyl alkanol derivative of general formula: 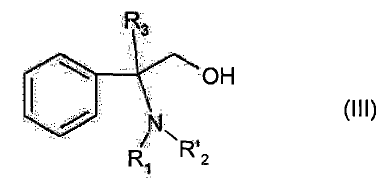 in which R₁ and R₃ are defined as previously and R'₂ is a hydrogen atom or is defined as R₂ in claim 1, then, if appropriate, when one of R'₂ or R₁ is the hydrogen atom, carries out the substitution of the amine of the 2-amino-2-phenyl-alkanol ester derivative obtained, of general formula: 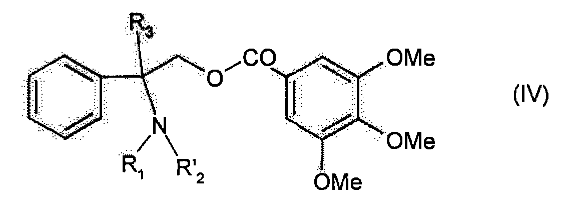 in which R₁ and R'₂ and R₃ are defined as above.
• either, when R'₂ is H, and if it is desired to obtain derivatives for which R₂ is -CO-R, by the action of a reactive derivative of the acid of general formula
R-COOH (V)
in which R is as defined in claim 1,
• or, when R'₂ is H, and if it is desired to obtain derivatives for which R₂ is -CO-Y-R₄, Y being O, S, NH or Nalk
- either, by the action of phosgene, followed by reaction with the alcohol, the thiol or the amine of general formula:
R₄-YH (VI)
in which R₄ is an optionally substituted alkyl radical and where if appropriate the functions in the reaction which can be altered are protected beforehand, or an aryl, aralkyl or heterocyclylalkyl radical, and Y is the oxygen or sulphur atom, or an NH or Nalk radical
- or, by the action of the halide of general formula:
R₄-Y-COHal (VII)
in which R₄ is defined as in claim 1, preferably branched aryl or alkyl, Y is the oxygen or sulphur atom and Hal is a halogen atom, preferably chlorine,
- or, when it is desired to obtain an R₄ radical bearing the -C(alk)-O-CO-R₅ substitution for which alk is defined as in claim 2 and R₅ is defined as in claim 1, by the action of chloroalkylchloroformate, followed by reacting the product obtained with an alkaline salt of the corresponding acid R₅COOH, or also the silver salt or a quaternary ammonium salt of this acid:
• or, when R'₂ is H, and if it is desired to obtain derivatives for which R₂ is substituted alkyl, or when a derivative of general formula (IV) has been obtained, for which R₁ is a hydrogen atom and R'₂ is defined as R₂ in claim 1, and if it is desired to obtain a product of general formula (I) for which R₁ is optionally substituted alkyl, by acylation by an acid halide or a reactive ester of structure:
R₂-CO-Z (VIIa)
or
R₁-CO-Z (VIIIb)
in which R₁ or R₂ are defined as above and Z is a halogen atom or the residue of a reactive ester, followed by reduction of the amide formed into an amine.
• or also, when a derivative of general formula (IV) has been obtained for which R₁ is a hydrogen atom and R'₂ is defined as R₂ in claim 1, and if it is desired to obtain a compound of general formula (I) for which R₁ is alkyl, by the action of a halogenated derivative on formula
R₁-X (IX)
in which R₁ is an alkyl radical and X is a halogen atom or a sulphonic radical, in the presence of a base,
then optionally the product obtained is converted to a pharmaceutically acceptable salt, When these exist.

6. Pharmaceutical composition comprising at least one product according to claim 1, in the purestate or in the form of a combination with one or more compatible and phamaceutically acceptable diluents or adjuvants.

## Patentansprüche

1. 2-Amino-2-phenylalkanolesterderivat mit der allgemeinen Formel: 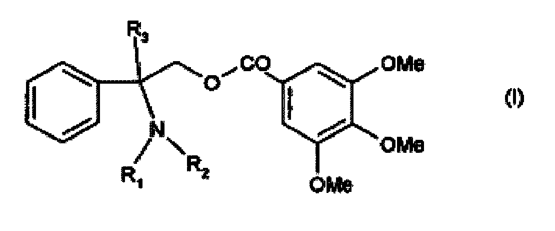 worin:
R₁ ein Wasserstoffatom, ein Alkylradikal, enthaltend 1 bis 4 geradkettige oder verzweigte Kohlenstoffatome, ein Alkylradikal, enthaltend 2 bis 4 geradkettige oder verzweigte Kohlenstoffatome, substituiert durch Hydroxy, Alkoxy, Alkylthio, Acyloxy, Amino, Alkylamino, Dialkylamino, Alkylcarbamoyloxy, Alkoxycarbonylamino, Ureido oder Alkylureido, ist,
R₂ ein Radikal -CO-R, worin R ein Alkylradikal, ein Aryl-, Heterocyclyl-, Benzyl- oder Heterocyclylmethylradikal,
ein Radikal -CO-Y-R₄ ist, wobei Y ein Heteroatom ist, ausgewählt aus -O-, -S-, -NH-, -Nalk-, wobei alk ein lineares oder verzweigtes Alkylradikal, enthaltend 1 bis 4 Kohlenstoffatome, ist und R₄ ausgewählt ist aus den Alkyl-, Aryl-, Aralkyl- oder Heterocyclylalkylradikalen, die durch ein oder mehrere Halogenatome oder Hydroxy-, Alkylradikale, enthaltend 1 bis 4 geradkettige oder verzweigte Kohlenstoffatome, Alkoxy-, Alkylthio-, Acylaminoalkylthio-, Alkoxycarbonyl- oder Acylaminoradikale, wovon die Alkylreste 1 bis 4 geradkettige oder verzweigte Kohlenstoffatome enthalten, oder oxo-Radikale substituiert sein können, oder durch ein Radikal R₅COO- substituiert sein können, worin R₅ für ein Alkylradikal steht, das gegebenenfalls durch Benzyloxycarbonylamino, Acylamino oder durch den Rest einer Aminosäure substituiert ist, oder für ein Heterocyclylradikal steht, oder auch
R₂ ein Alkylradikal ist, enthaltend 2 bis 4 Kohlenstoffatome, das durch Hydroxy, Alkoxy, Alkylthio, Acyloxy, Amino, Alkylamino, Dialkylamino substituiert ist, wovon die Alcoylanteile mit dem Stickstoffatom, an das sie gebunden sind, einen Heterozyklus mit 5 oder 6 Gliedern bilden können, der gegebenenfalls ein anderes Heteroatom trägt, das aus Sauerstoff und Stickstoff ausgewählt ist, oder mit Alkylcarbamoyloxy, Alkoxycarbonylamino, Ureido oder Alkylureido substituiert ist, wobei es sich versteht, dass das substituierte Alkylradikal geradkettig oder verzweigt ist und mindestens 2 Kohlenstoffe zwischen dem Stickstoffatom, das R₂ trägt, und dem Substituenten aufweist;
R₃ ein Alkylradikal ist, enthaltend 1 bis 4 geradkettige oder verzweigte Kohlenstoffatome in ihren R- oder S-Formen oder deren Gemischen sowie, falls es sie gibt, ihren pharmazeutisch akzeptablen Salzen.

2. 2-Amino-2-phenylalkanolesterderivat nach Anspruch 1, **dadurch gekennzeichnet, dass** es der allgemeinen Formel (I) entspricht, wobei R₁ und R₃ sind, wie in Anspruch 1 definiert, und R₂ in -NR₁R₂ ein Radikal -CO-O-R₄ ist, wobei R₄ eine Struktur:
C(alk)-O-CO-R₅ (X)
aufweist, wobei alk ein Alcoylradikal ist, enthaltend 1 bis 6 geradkettige oder verzweigte Kohlenstoffatome, und R₅ ist, wie in Anspruch 1 definiert, wobei der Ester mit (Ia) bezeichnet wird, oder auch **dadurch gekennzeichnet, dass** er der allgemeinen Formel (I) entspricht, wobei R₃ ist, wie in Anspruch 1 definiert, und R₁ in -NR₁R₂ ein Wasserstoffatom ist und R₂ ein Radikal -CO-R ist, wie in Anspruch 1 definiert, wobei der Ester mit (Ia') bezeichnet wird.

3. 2-Amino-2-phenylalkanolesterderivat nach Anspruch 2, bezeichnet mit (Ia), **dadurch gekennzeichnet, dass** es der allgemeinen Formel (I) entspricht, wobei R₁ ein Wasserstoffatom, ein Alkylradikal, enthaltend 1 bis 4 Kohlenstoffatome, bevorzugt Methyl, oder ein 2-Methoxyethylradikal ist, und R₂ und R₃ sind, wie in Anspruch 2 für den Ester (Ia) definiert.

4. 2-Amino-2-phenylalkanolesterderivat nach Anspruch 1, **dadurch gekennzeichnet, dass** es der allgemeinen Formel (I) entspricht, wobei R₁ und R₃ sind, wie in Anspruch 1 definiert, und R₂ in -NR₁R₂ ein substituiertes Alkylradikal ist, wie für R₂ in Anspruch 1 definiert, wobei der Ester mit (Ib) bezeichnet wird.

5. Verfahren zur Herstellung eines Derivats nach Anspruch 1, **dadurch gekennzeichnet, dass** ein Derivat mit der allgemeinen Formel: 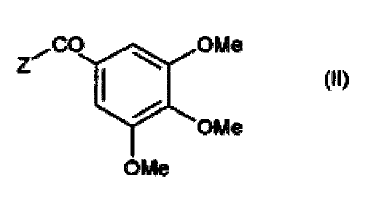 worin Z ein Halogenatom, ein Hydroxyradikal oder der Rest eines reaktiven Esters ist, mit dem 2-Amino-2-phenylalkanolester mit der allgemeinen Formel: 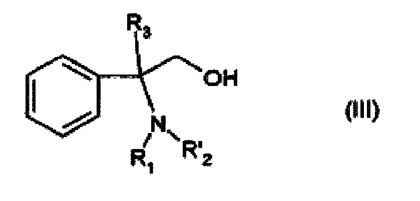 worin R₁ und R₃ sind, wie zuvor definiert und R'₂ ein Wasserstoffatom ist, oder wie R₂ in Anspruch 1 definiert ist, umgesetzt wird, dann gegebenenfalls, wenn eines von R'₂ oder R₁ das Wasserstoffatom ist, die Substitution des Amins des erhaltenen 2-Amino-2-phenylalkanolesterderivats mit der allgemeinen Formel: 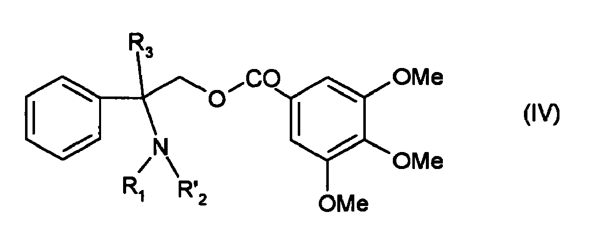 worin R₁, R'₂ und R₃ sind, wie oben definiert, durchgeführt wird,
• entweder, wenn R'₂ H ist, und wenn man Derivate erhalten will, für die R₂ -CO-R ist, durch Einwirkung eines reaktiven Derivats der Säure mit der allgemeinen Formel
R-COOH (V)
worin R definiert ist wie in Anspruch 1,
• oder, wenn R'₂ H ist, und wenn man Derivate erhalten will, für die R₂ -CO-Y-R₄ ist, wobei Y O, S, NH oder Nalk ist,
- entweder durch die Einwirkung von Phosgen, gefolgt von der Umsetzung mit Alkohol, Thiol oder dem Amin mit der allgemeinen Formel:
R₄-YH (VI)
worin R₄ ein gegebenenfalls substituiertes Alkylradikal ist, und dessen, bei der Umsetzung gegebenenfalls veränderbare Funktionen, vorher geschützt werden, oder ein Aryl-, Aralkyl- oder Heterocyclylalkylradikal, und Y das Sauerstoff- oder Schwefelatom oder ein Radikal NH oder Nalk ist,
- oder durch Einwirkung des Halogenids mit der allgemeinen Formel:
R₄-Y-COHal (VII)
worin R₄ ist, wie in Anspruch 1 definiert, bevorzugt verzweigtes Aryl oder Alkyl, Y das Sauerstoff- oder Schwefelatom ist und Hal ein Halogenatom, bevorzugt Chlor, ist,
- oder, wenn man ein Radikal R₄ erhalten will, das die Substitution -C(alk)-O-CO-R₅ trägt, wobei alk ist, wie in Anspruch 2 definiert, und R₅ ist, wie in Anspruch 1 definiert, durch Einwirkung von Chloralkylchlorformiat, gefolgt von der Umsetzung des erhaltenen Produkts mit einem alkalischen Salz der entsprechenden Säure R₅COOH oder auch dem Silbersalz oder einem quartären Ammoniumsalz dieser Säure,
• oder, wenn R'₂ H ist, und wenn man Derivate erhalten will, für die R₂ ein substituiertes Alkyl ist, oder auch, wenn man ein Derivat mit der allgemeinen Formel (IV) erhalten hat, wobei R₁ ein Wasserstoffatom ist und R'₂ definiert ist, wie R₂ in Anspruch 1, und wenn man ein Produkt mit der allgemeinen Formel (I) erhalten will, wobei R₁ gegebenenfalls substituiertes Alkyl ist, durch Acylierung mit einem Säurehalogenid oder einem reaktiven Ester mit der Struktur:
R₂-CO-Z (VIIIa)
oder
R₁-CO-Z (VIIIb)
worin R₁ oder R₂ sind, wie oben definiert, und Z ein Halogenatom oder der Rest eines reaktiven Esters ist, gefolgt von der Reduktion des gebildeten Amids in ein Amin,
• oder auch, wenn man ein Derivat mit der allgemeinen Formel (IV) erhalten hat, wobei R₁ ein Wasserstoffatom ist, und R'₂ ist, wie R₂ in Anspruch 1 definiert, und wenn man ein Produkt mit der allgemeinen Formel (I) erhalten will, wobei R₁ Alkyl ist, durch Einwirkung eines Halogenderivats mit der Formel
R₁-X (IX)
worin R₁ ein Alkylradikal ist und X ein Halogenatom oder ein Sulfonradikal ist, in Gegenwart einer Base,
dann das erhaltene Produkt gegebenenfalls in ein pharmazeutisch akzeptables Salz, falls es eines gibt, überführt.

6. Pharmazeutische Zusammensetzung, umfassend mindestens ein Produkt nach Anspruch 1 in reinem Zustand oder in Form einer Assoziation mit einem oder mehreren Verdünnungsmitteln oder Adjuvanzien, die kompatibel und pharmazeutisch akzeptabel sind.
